# EUROPEAN PATENT SPECIFICATION

(11) **EP 1 603 544 B1**
(45) Date of publication and mention of the grant of the patent: **10.10.2007**
(21) Application number: 04720110.8
(22) Date of filing: 12.03.2004
(51) Int. Cl.: A61K 31/135, A61P 1/12

(54) **TREATMENT OF INTESTINAL CONDITIONS WITH N-2,3,3-TETRAMETHYLBICYCLO 2.2.1 HEPTAN-2-AMINE**
BEHANDLUNG VON GASTROINTESTINALEN ERKRANKUNGEN MIT N-2,3,3-TETRAMETHYLBICYCLO¬2.2.1|HEPTAN-2-AMINE
TRAITEMENT DE PROBLEMES INTESTINAUX AVEC N-2,3,3-TETRAMETHYLBICYCLO 2.2.1|HEPTAN-2-AMINE

(30) Priority: 14.03.2003 US 454527 P
(43) Date of publication of application: 14.12.2005
(73) Proprietor: AGI Therapeutics Research Limited, Dublin 8 (IE)
(72) Inventor: DEVANE, John, Athlone, Co. Roscommon (IE)
(74) Representative: MacLean, Martin Robert
(86) International application number: PCT/IB2004/001134
(87) International publication number: WO 2004/080446

(56) References cited:
- WO-A-00/33812
- WO-A-00/35279
- WO-A-00/35280
- MANGEL A W: "Potentiation of colonic contractility to cholecystokinin and other peptides." EUROPEAN JOURNAL OF PHARMACOLOGY. 4 MAY 1984, vol. 100, no. 3-4, 4 May 1984 (1984-05-04), pages 285-290, XP002287147 ISSN: 0014-2999
- BRAIDA D ET AL: "Excitatory and inhibitory effects of second-generation cholinesterase inhibitors on rat gastrointestinal transit." PHARMACOLOGICAL RESEARCH : THE OFFICIAL JOURNAL OF THE ITALIAN PHARMACOLOGICAL SOCIETY. JUN 2000, vol. 41, no. 6, June 2000 (2000-06), pages 671-677, XP002287148 ISSN: 1043-6618

## Description

This application claims the benefit of U.S. Provisional Application No. 60/454,527, filed March 14, 2003.

The present invention comprises methods and formulations for reducing, preventing, and/or managing abnormal increases in gastrointestinal motility. Such abnormal increases can be caused by one or more intestinal conditions, including, but not limited to functional bowel disorders including irritable bowel syndrome (IBS), functional abdominal bloating, functional diarrhea, and unspecified functional bowel disorder, inflammatory bowel disease (IBD); ulcerative colitis; granulomatous enteritis; Crohn's disease; infectious diseases of the small and/or large intestine; pyloric spasm; abdominal cramps; mild dysenteries; diverticulitis; acute enterocolitis; neurogenic bowel disorders; the splenic flexure syndrome; neurogenic colon; spastic colitis; and/or symptoms of any of the foregoing. The present invention comprises methods and formulations for reducing, preventing, and/or managing abnormal increases in gastrointestinal motility with ganglionic blocking agents and/or nicotinic-receptor antagonists including, but not limited to, N-2,3,3-tetramethylbicyclo-[2.1.1]heptan-2-amine, and pharmaceutically acceptable salts thereof.

Gastrointestinal conditions pose a significant worldwide health problem. Functional bowel disorders are functional gastrointestinal disorders with symptoms attributable to the mid or lower gastrointestinal tract, including irritable bowel syndrome (IBS), functional abdominal bloating, functional constipation, functional diarrhea, and unspecified functional bowel disorder. These disorders are identified by symptoms according to the diagnostic criteria that were revised by the 1998 Working Team (Thompson et al., Gut, 45 (Suppl II): 1143-1147, 1999). The diagnosis of a functional bowel disorder requires symptoms for 12 weeks or more within the past 12 months (although it is not necessary that the 12 weeks be consecutive) and presumes the absence of a structural or biochemical explanation for the symptoms.

For example, irritable bowel syndrome results in about 3.5 million physician visits per year, and is the most common diagnosis made by gastroenterologists, accounting for about 25% of all subjects diagnosed (Camilleri and Choi, Aliment Pharmacol. Ther., 11(1):3-15, 1997). Subjects afflicted with IBS visit doctors more frequently, enjoy a lower quality of life, and miss work more often relative to those with no bowel symptoms (Drossman et al, Dig. Dis. Sci., 38:1569-1580, 1993). As a consequence, individuals suffering from IBS incur significantly higher health care costs than those without the condition (Talley et al., Gastroenterology, 109:1736-1741, 1995).

IBS is diagnosed by abdominal discomfort or pain that includes two of the following three features: (1) relieved with defecation, (2) onset associated with a change in frequency of stool, and/or (3) onset associated with a change in form (appearance) of stool for at least 12 weeks, which need not be consecutive, in the past 12 months (Thompson *et al.,* 1999). IBS is associated with abdominal discomfort or pain and altered bowel function (Caner et al., Gastroenterology, 107:271-93, 1994; Camilleri and Choi, 1997; Drossman et al., Am. J. Gastroent., 91:2270-81, 1996). The condition leads to crampy pain, gassiness, bloating, and changes in bowel habits. Some subjects with IBS have constipation (difficult or infrequent bowel movements); others have diarrhea (frequent loose stools, often with an urgent need to move the bowels); and some experience both. Sometimes a subject with IBS has a crampy urge to move the bowels but cannot do so. For a general description of IBS, see, *e.g.,* NIH Publication No. 97-693, National Digestive Diseases Information Clearinghouse, National Institute of Health, 1992 and NIH Publication No. 03-693, National Digestive Diseases Information Clearinghouse, National Institute of Health, April 2003.

IBS is a well-recognized clinical entity, but no causative etiologic agents or structural or biochemical defects have been positively identified. In many subjects, intraluminal contents exhibit unusually rapid transit through the length of the small intestine and colon. Afflicted subjects generally complain of abdominal discomfort and report audible bowel noises, cramping and abdominal pain, an urgency to defecate, and the passage of loose stools often covered with mucus.

Functional diarrhea refers to continuous or watery stools without abdominal pain. The diagnostic criteria include liquid (mushy) or watery stools that are present 75% of the time in the absence of abdominal pain (Thompson *et al.,* 1999). Diagnostic criteria for other functional bowel disorders are described in Thompson *et al.,* 1999 as well.

Inflammatory diseases of the bowel are commonly classified under the generic term of inflammatory bowel diseases (IBD), which encompasses a range of diseases including, but not limited to, Crohn's disease and ulcerative colitis. IBD affects nearly 1 million people in the United States.

Currently, among the treatment options for subjects suffering from IBD are 5-aminosalicylic acid and its congeners, various steroids, azathioprene and 6-mercaptopurine, cyclosporine, methotrexate, fish oils, remicade, heparin, antimicrobials, such as ciprofloxacin and metronidazole, and nicotine (Wolf and Lashner, Cleveland Clinic Journal of Medicine, 69(8), 621-631, 2002). However, each of these therapies is associated with potential adverse effects that can occasionally be severe. Therefore, subjects suffering from ailments included in the IBD family are in need of effective treatments that exhibit reduced side effects.

Recently, a new nicotinic-receptor agonist, TC-2403-12, reportedly entered clinical trials for the treatment of ulcerative colitis (Targacept press release entitled "Targacept and Dr. Falk Pharma Successfully Complete Phase I Studies of First in Class Drug for Ulcerative Colitis," Winston-Salem, NC, October 1, 2002). In general, it is thought that nicotinic agonists can be helpful in alleviating inflammatory conditions of the gastrointestinal tract. They are not thought, however, to have a direct effect on the motility of the gastrointestinal tract, as contemplated by the methods and formulations of the present invention.

To date, no effective, long-term treatments for IBS or IBD have been identified. Accordingly, an urgent need exists for new treatments of IBS and IBD that are both effective and improve the subject's quality of life.

Mecamylamine HCl (*N*-2,3,3-tetramethytbicyclo[2.2.1]heptan-2-amine hydrochloride) is a ganglionic blocking agent (Stone et al., J. Pharm. Exp. Ther., 117(2), 169-183, 1956; Stone et al., J. Med Pharm. Chem., 5(4), 655-90, 1962). It is also recognized to cross the blood-brain barrier and function as a selective nicotinic-receptor antagonist (Papke et al., J. Pharmacol. Exp. Ther., 297(2), 646-656, 2001). The compound has been used as a treatment for cardiovascular conditions, such as hypertension (Stone et al., British Med. J., No. 5016, 422-425, 1957). It has also been used in the treatment of autonomic dysreflexia (Braddom et al., Am. J. Phys. Med. Rehabil., 70(5), 234-240, 1991), as an aid in smoking cessation (Stolerman et al., Pyschopharmacoliga, 28, 247-259, 1973; Tennant et al., NIDA Res. Monograph, 291-297, 1984; Rose et al., Clin. Pharm. Ther., 56(1), 86-99, 1994; Rose et al., Exp. Clin. Pharmacol., 6(3), 331-343, 1998; Zevin et al., Clin Pham. & Therapeutics, 68(1), 58-66, 2000; and WO 0033812), as an aid in decreasing the dependence on cocaine (Reid et al., Neuropsychopharmacology, 20(3), 297-307, 1999), and has been investigated in the treatment of certain CNS conditions, such as Tourette's syndrome (Sandberg et al., Lancet, 352(9129), 705-706, 1998; Young et al., Clinical Therapeutics, 23(4), 2001; Silver et al., Child and Adolescent Psych., 40(9), 1103-1110, 2001).

Additionally, United States Patent Application Publications 2002/0016370 and 2002/0016371 disclose the use of *exo*-(R)-*N*-2,3,3-tetramethylbicyclo[2.2.1]heptan-2-amine, or a pharmaceutically acceptable salt thereof, and *exo*-(S)-*N*-2,3,3-tetramethylbicyclo[2.2.1]heptan-2-amine, or a pharmaceutically acceptable salt thereof, respectively, for use in the treatment of medical conditions such as substance addiction, smoking cessation, hypertension, hypertensive crises, Tourette's syndrome and other tremors, cancer, atherogenic profile, neuropsychiatric disorders, chronic fatigue syndrome, Crohn's disease, autonomic dysreflexia, and spasmogenic intestinal disorders.

It is known that when mecamylamine hydrochloride is dosed orally using conventional formulations, the compound is nearly completely and rapidly absorbed from the gastrointestinal tract, leading to rapid attainment of a maximum plasma concentration (Bear et al., Am. J. Physiol., 186, 180-6, 1956). For example, Young *et al.* report that the administration of a 2.5 mg dose of mecamylamine hydrochloride to adults in a conventional formulation provides a maximum plasma concentration (Cmax) of about 7.89 ng/mL and a time to achieve the highest plasma concentration (Tmax) of 3.11 hours. Additionally, a 7.5 mg dose of mecamylamine hydrochloride to adults in a conventional formulation provides a Cmax of 23.68 ng/mL and a Tmax of 3.04 hours, so that the pharmacokinetic parameters are reported to be dose-proportional. (Young et al., Clinical Therapeutics, 23(4), 2001). This report also shows that the average half-life of elimination of mecamylamine, dosed using conventional formulations, is about 10.1 hours to about 10.5 hours at either the 2.5 mg or 7.5 mg dose level. Although not reported by Young *et al.,* one skilled in the art can calculate from this data that the expected ratio of peak plasma concentration of mecamylamine to plasma concentration of mecamylamine 24 hours after dosing would be about 4:1. Furthermore, it is expected that about 50% of the peak plasma concentration of mecamylamine would be maintained for about 14 hours, with the 24 hour plasma concentration level being less than about 25% of peak plasma concentration. The typical dose used for treating hypertensive subjects is about 25 mg/day, and is dosed using conventional formulations. From this dose, one skilled in the art would expect that the peak plasma concentration of mecamylamine would be about 78.9 ng/mL. The once-daily administration of mecamylamine in this manner provides plasma concentration levels that can cause undesirable side-effects, including impaired sexual function, cycloplegia, xerostomia, diminished perspiration, postural hypotension, hypothermia, tremors, anti-diuresis, antinociception, blurred vision, impotency, dysuria, tremor, choreiform movements, mental aberrations, nervousness, depression, anxiety, insomnia, slurred speech, weakness, fatigue, sedation, headache, constipation and renal insufficiency (Young et al., Clinical Therapeutics, 23(4), 2001). WO 00/35279 and WO 00/35280 also describe compositions containing mecamylamine.

The present invention relates to use of N-2,3,3-tetramethylbicyclo-[2.1.1]heptan-2-amine, or a pharmaceutically acceptable salt thereof for the manufacture of a medicament for reducing gastrointestinal motility in a subject suffering from an abnormal increase in gastrointestinal motility wherein said N-2,3,3-tetramethylbicyclo-[2.1.1]heptan-2-amine comprises racemic N-2,3,3-tetramethylbicyclo-[2.1.1]heptan-2-amine, enriched (R)-N-2,3,3-tetramethylbicyclo-[2.1.1]heptan-2-amine, or enriched (S)-N-2,3,3-tetramethylbicyclo-[2.1.1]heptan-2-amine, and wherein said abnormal increase in gastrointestinal motility is caused by at least one condition chosen from inflammatory bowel disease, ulcerative colitis, granulomatous enteritis, infectious diseases of the small or large intestine, pyloric spasm, abdominal cramps, a functional bowel disorder, mild dysenteries, diverticulitis, acute enterocolitis, neurogenic bowel disorders, splenic flexure syndrome, neurogenic colon, spastic colitis, or is a symptom of any of the foregoing conditions. The present invention further relates to pharmaceutically acceptable formulations comprising N-2,3,3-tetramethylbicyclo[2.2.1]heptan-2-amine, or a pharmaceutically acceptable salt thereof, that when administered to a subject decrease abnormal gastrointestinal motility while also decreasing the incidence of side effects as compared to the administration of mecamylamine in conventional formulations.

Such a decrease in side effects, in accordance with this invention, can be achieved, for example, by the administration of *N*-2,3,3-tetramethylbicyclo[2.2.1]heptan-2-amine, or a pharmaceutical acceptable salt thereof, in the form of a modified-release formulation. The administration of N-2,3,3-tetramethylbicyclo[2.2.1]heptan-2-amine in this manner can provide a plasma concentration of N-2,3,3-tetramethylbicyclo[2.2.1]heptan-2-amine in a subject that is relatively constant and avoids the high initial concentrations associated with the administration of N-2,3,3-tetramethylbicyclo[2.2.1]heptan-2-amine in conventional formulations. Relatively constant plasma concentrations of N-2,3,3-tetramethylbicyclo[2.2.1]heptan-2-amine benefit the subject by decreasing abnormal gastrointestinal motility while also decreasing unwanted side effects associated with the relatively high initial plasma concentrations of N-2,3,3-tetramethylbicyclo[2.2.1]heptan-2-amine observed when it is administered in conventional formulations. Additionally, such modified-release formulations allow dosage administration at a reduced frequency, such as once daily. This frequency of dosing can increase the convenience for subjects using N-2,3,3-tetramethylbicyclo[2.2.1]heptan-2-amine, or a pharmaceutically acceptable salt thereof, for the intended use and can also improve subject compliance with dosing instructions.

The present invention pharmaceutically acceptable formulations comprising N-2,3,3-tetramethylbicyclo[2.2.1]heptan-2-amine, or a pharmaceutically acceptable salt thereof, in the form of a modified-release or a combined modified-release and immediate-release formulation.

The present invention also provides that such modified-release or combined modified-release and immediate-release formulations comprising N-2,3,3-tetramethylbicyclo[2.2.1]heptan-2-amine, or a pharmaceutically acceptable salt thereof, can be in several forms, including, but not limited to, those suitable for oral, intra-nasal, buccal, sublingual, and transdermal administration.

The present invention also includes the use of a gastrointestinal motility decreasing amount of N-2,3,3-tetramethylbicyclo[2.2.1]heptan-2-amine, or a pharmaceutically acceptable salt thereof, in the form of a pharmaceutically acceptable modified-release or combined modified-release and immediate-release formulation for the manufacture of a medicament for reducing gastrointestinal motility in a subject suffering from an abnormal increase in gastrointestinal motility.

The present invention also provides formulations for achieving a maximum plasma concentration of N-2,3,3-tetramethylbicyclo[2.2.1]heptan-2-amine in a subject at about 3.5 hours, or later, following any administration according to the invention. The maximum plasma concentration of N-2,3,3-tetramethylbicyclo[2.2.1]heptan-2-amine may be maintained for about 3.5 hours to about 7 days after administration. The present formulations provide the benefit of decreasing intestinal motility in a subject in need of such reduction, while reducing at least one side effect associated with the administration of N-2,3,3-tetramethylbicyclo[2.2.1]heptan-2-amine in conventional formulations. Such effects may be effects on the subject's heart rate, blood pressure, vision, and bladder function.

Additionally, the formulations of the invention provide a peak:trough plasma level ratio of N-2,3,3-tetramethylbicyclo[2.2.1]heptan-2-amine of less than about 4:1, in some embodiments less than about 3:1, and in some embodiments less than about 2:1. As used herein with reference to peak:trough ratios, "peak" means the maximum plasma concentration, or Cmax, and "trough" means the plasma level at 24 hours following a first administration, and during which said 24-hour period only one administration of a formulation of the invention is given to a subject.

Additionally, the formulations of the invention provide a plasma concentration of N-2,3,3-tetramethylbicyclo[2.2.1]heptan-2-amine, at least about 24 hours following a first administration, that is greater than or equal to at least about 25%, 30%, 35%, 40%, 45%, 50%, 60%, 70%, 80%, 90%, 100% in various embodiments, or any number in between, of the peak plasma concentration achieved following administration.

Additionally, the formulations of the invention provide a plasma concentration of N-2,3,3-tetramethylbicyclo[2.2.1]heptan-2-amine that is greater than or equal to about 50% of the peak plasma concentration for greater than or equal to about 14 hours, 16 hours, 18 hours, 21 hours, 24 hours, or any number in between, or longer, following a first administration.

Additionally, the present invention provides the use of a gastrointestinal motility reducing amount of racemic N-2,3,3-tetramethylbicyclo-[2.1.1]heptan-2-amine, enriched (R)-N-2,3,3-tetramethylbicyclo-[2.1.1]heptan-2-amine, enriched (S)-N-2,3,3-tetramethylbicyclo-[2.1.1]heptan-2-amine, substantially pure (R)-N-2,3,3-tetramethylbicyclo-[2.1.1]heptan-2-amine, or substantially pure (S)-N-2,3,3-tetramethylbicyclo-[2.1.1]heptan-2-amine, or pharmaceutically acceptable salts thereof. For the manufacture of a medicament for reducing gastrointestinal motility in a subject suffering from an abnormal increase in gastrointestinal motility. In one embodiment, the medicament comprises racemic N-2,3,3-tetramethylbicyclo-[2.1.1]heptan-2-amine, or a pharmaceutically acceptable salt thereof.

The present invention also provides pharmaceutical formulations comprising racemic N-2,3,3-tetramethylbicyclo-[2.1.1]heptan-2-amine, enriched (R)-N-2,3,3-tetramethylbicyclo-[2.1.1]heptan-2-amine, enriched (S)-N-2,3,3-tetramethylbicyclo-[2.1.1]heptan-2-amine, substantially pure (R)-N-2,3,3-tetramethylbicyclo-[2.1.1]heptan-2-amine, or substantially pure (S)-N-2,3,3-tetramethylbicyclo-[2.1.1]heptan-2-amine or pharmaceutically acceptable salts thereof, in the form of a modified-release or a combined modified-release and immediate-release formulation. In one embodiment, the formulation comprises racemic N-2,3,3-tetramethylbicyclo-[2.1.1]heptan-2-amine, or a pharmaceutically acceptable salt thereof.

The present inventive formulations provide modified-release formulations comprising N-2,3,3-tetramethylbicyclo[2.2.1]heptan-2-amine, or a pharmaceutically acceptable salt thereof, in which less than about 50% of said N-2,3,3-tetramethylbicyclo[2.2.1]heptan-2-amine is released in vitro in less than about 2 hours, greater than or equal to about 40% is released in about 12 or more hours, and about 70% or more is released in about 24 or more hours.

Oral formulations according to the present invention, when measured by a U.S. Pharmacopoeia (USP) Type 1 Apparatus (baskets) or U.S. Pharmacopeia (USP) Type 2 Apparatus (paddles) at 37°C and 50 rpm or higher in phosphate buffer at pH 6.8 or higher for the measuring period, can exhibit the following dissolution profile: 2 hours: less than or equal to about 50%; 4 hours: less than or equal to about 70%; 8 hours: greater than or equal to about 50%; 12 hours: greater than or equal to about 65%; and 24 hours: greater than or equal to about 80%. In other embodiments, the oral formulation can exhibit the following profile: 2 hours: less than or equal to about 40%; 4 hours: less than or equal to about 65%; 8 hours: greater than or equal to about 60%; 12 hours: greater than or equal to about 70%; and 24 hours: greater than or equal to about 80%. In still other embodiments, the oral formulation can exhibit the following profile: 2 hours: less than or equal to about 30%; 4 hours: about 20% to about 60%; 8 hours: greater than or equal to about 70%; 12 hours: greater than or equal to about 75%; and 24 hours: greater than or equal to about 80%.

Transdermal formulations according to the present invention, when tested using modified Franz diffusion cells of human epidermis (according to methods adapted from Franz, J. Invest. Dermatol. 64:194-195 (1975) and GB-A-2 098 865), in ammonium phosphate buffer at pH 4.0 or higher, while stirring the receiving compartment, at 300 RPM for example, and maintaining the temperature at 32 °C for the duration of the study, can exhibit the following dissolution profile: 2 hours: less than or equal to about 40%; 4 hours: about 10% to about 70%; 8 hours: about 20% to about 80%; 12 hours: greater than or equal to about 40%; and 24 hours: greater than or equal to about 70%. In other embodiments, the transdermal formulation can exhibit the following profile: 2 hours: less than or equal to about 30%; 4 hours: about 15% to about 60%; 8 hours: about 30% to about 70%; 12 hours: greater than or equal to about 50%; and 24 hours: greater than or equal to about 75%. In still other embodiments, the transdermal formulation can exhibit the following profile: 2 hours: less than or equal to about 25%; 4 hours: about 20% to about 50%; 8 hours: about 40% to about 70%; 12 hours: greater than or equal to about 55%; and 24 hours: greater than or equal to about 80%.

The present invention also provides the use of a gastrointestinal motility decreasing amount of racemic N-2,3,3-tetramethylbicyclo-[2.1.1 ]heptan-2-amine, enriched (R)-N-2,3,3-tetramethylbicyclo-[2.1.1]heptan-2-amine, substantially pure (R)-N-2,3,3-tetramethylbicyclo-[2.1.1]heptan-2-amine, enriched (S)-N-2,3,3-tetramethylbicyclo-[2.1.1]heptan-2-amine, or substantially pure (S)-N-2,3,3-tetramethylbicyclo-[2.1.1]heptan-2-amine, or pharmaceutically acceptable salts thereof, in the form of a pharmaceutically acceptable modified-release, or a combined modified-release and immediate-release formulation, for the manufacture of a medicament for reducing gastrointestinal motility in a subject suffering from an abnormal increase in gastrointestinal motility. Such formulations can be in a form suitable for, but not limited to oral, intra-nasal, buccal, sublingual, or transdermal administration. In one embodiment, the medicament comprises racemic N-2,3,3-tetramethylbicyclo-[2.1.1]heptan-2 amine, or a pharmaceutically acceptable salt thereof.

The present invention overcomes the deficiencies and problems in the prior art by providing new and effective formulations for reducing, preventing, and/or managing abnormal increases in gastrointestinal motility, intestinal conditions that cause the same, and symptoms thereof.

The abnormal increases in gastrointestinal motility can be caused by one or more intestinal conditions. Thus, the present invention can also be used to directly or indirectly reduce, prevent, and/or manage such intestinal conditions by decreasing gastrointestinal motility. Examples of intestinal conditions that can be treated, prevented, and/or managed according to the present invention include, but are not limited to, functional bowel disorders including irritable bowel syndrome (IBS), functional abdominal bloating, functional diarrhea, and unspecified functional bowel disorder; inflammatory bowel disease (IBD); ulcerative colitis; granulomatous enteritis; Crohn's disease; infectious diseases of the small and large intestine; pyloric spasm; abdominal cramps; mild dysenteries; diverticulitis; acute enterocolitis; neurogenic bowel disorders including the splenic flexure syndrome and neurogenic colon; spastic colitis; and/or symptoms of any of the foregoing. Those of ordinary skill in the art will be familiar with other types of intestinal conditions that produce abnormal increases in gastrointestinal motility, which can benefit from the present invention.

As used herein, the term "an abnormal increase in gastrointestinal motility" means the circumstances in which a subject experiences an increase in the motility of the gastrointestinal tract due to a disease or an abnormal condition. Those conditions include, but are not limited to, diseases that are currently recognized such as functional bowel disorders including irritable bowel syndrome (IBS), functional abdominal bloating, functional diarrhea, and unspecified functional bowel disorder; inflammatory bowel disease (IBD); ulcerative colitis; granulomatous enteritis; Crohn's disease; infectious diseases of the small and large intestine; pyloric spasm; abdominal cramps; mild dysenteries; diverticulitis; acute enterocolitis; neurogenic bowel disorders including the splenic flexure syndrome and neurogenic colon; spastic colitis; and/or symptoms of any of the foregoing. Also included are those conditions or diseases which are currently unrecognized but display the same clinical symptoms of IBS, IBD, Crohn's disease, or ulcerative colitis, such as increases in gastrointestinal motility, abdominal discomfort and audible bowel noises, cramping and abdominal pain, an urgency to defecate, the passage of loose stools covered with mucus, and diarrhea. Changes in gastrointestinal motility according to the invention can be measured by any known method, for example the hydrogen breath test following lactulose administration according to Miller et al., Dig. Dis. Sci., 42:10-18, 1997.

As used herein, the term "N-2,3,3-tetramethylbicyclo[2.1.1]heptan-2-amine" encompasses a pure stereoisomer of N-2,3,3-tetramethylbicyclo[2.1.1]heptan-2-amine, such as pure *exo*-(R)-N-2,3,3-tetramethylbicyclo[2.1.1]heptan-2-amine, *exo*-(S)N-2,3,3-tetramethylbicyclo-[2.1.1]heptan-2-amine, *endo*-(R)-N-2,3,3-tetramethylbicyclo-[2.1.1]heptan-2-amine, and *endo-*(S)-N-2,3,3-tetramethylbicyclo-[2.1.1]heptan-2-amine, for example, or a mixture of any and all possible stereoisomers of N-2,3,3-tetramethylbicyclo-[2.1.1]heptan-2-amine, including *exo*-(R)-N-2,3,3-tetramethylbicyclo-[2.1.1]heptan-2-amine, *exo*-(S)-N-2,3,3-tetramethylbicyclo-[2.1.1]heptan-2-amine, *endo*-(R)-N-2,3,3-tetramethylbicyclo-[2.1.1]heptan-2-amine, and *endo-*(S)-N-2,3,3-tetramethylbicyclo-[2.1.1]heptan-2-amine, in any and all proportions, unless otherwise stated. Also included in this definition are mixtures of stereoisomers of N-2,3,3-tetramethylbicyclo-[2.1.1]heptan-2-amine in which two enantiomers, *exo*-(R)-N-2,3,3-tetramethylbicyclo-[2.1.1 ]heptan-2-amine and *exo*-(S)-N-2,3,3-tetramethylbicyclo-[2.1.1]heptan-2-amine for example, are present in equal amounts. Such mixtures are herein termed "racemic" compositions. Also included in this definition are mixtures comprising N-2,3,3-tetramethylbicyclo-[2.1.1]heptan-2-amine in which one stereoisomer is present in an amount greater than the others. For example, the mixture can comprise N-2,3,3-tetramethylbicyclo-[2.1.1]heptan-2-amine in which the *exo*-(R)-N-2,3,3-tetramethylbicyclo-[2.1.1]heptan-2-amine stereoisomer is present in an amount greater than the others. Such mixtures are herein termed "enriched (R)-N-2,3,3-tetramethylbicyclo-[2.1.1]heptan-2-amine" compositions. Alternatively, the mixture can comprise N-2,3,3-tetramethylbicyclo-[2.1.1]heptan-2-amine in which the *exo*-(S)-N-2,3,3-tetramethylbicyclo-[2.1.1]heptan-2-amine stereoisomer is present in an amount greater than the others. Such formulations are herein termed "enriched (S)-N-2,3,3-tetramethylbicyclo-[2.1.1]heptan-2-amine" compositions. In addition, an enriched mixture can comprise N-2,3,3-tetramethylbicyclo-[2.1.1]heptan-2-amine in which the *exo*-(R)-N-2,3,3-tetramethylbicyclo-[2.1.1]heptan-2-amine stereoisomer the predominant isomer, present in an amount greater than or equal to ninety percent more than the others. Such mixtures are herein termed "substantially pure (R)-N-2,3,3-tetramethylbicyclo-[2.1.1]heptan-2-amine" compositions. Alternatively, an enriched mixture can comprise N-2,3,3-tetramethylbicyclo-[2.1.1]heptan-2-amine in which the *exo-*(S)-N-2,3,3-tetramethylbicyclo-[2.1.1] heptan-2-amine stereoisomer is present in an amount greater than or equal to ninety percent more than the others. Such formulations are herein termed "substantially pure (S)-N-2,3,3-tetramethylbicyclo-[2.1.1]heptan-2-amine" compositions. One skilled in the art will appreciate that "enriched" *exo*-(S)-N-2,3,3-tetramethylbicyclo-[2.1.1]heptan-2-amine encompasses "substantially pure" *exo*-(S)-N-2,3,3-tetramethylbicyclo-[2.1.1]heptan-2-amine. It is also contemplated that N-2,3,3-tetramethylbicyclo-[2.1.1]heptan-2-amine can be present as one or more pharmaceutically acceptable salts in any formulation of the invention.

As used herein, the term "modified-release" formulation or dosage form includes pharmaceutical preparations that achieve a desired release of the drug from the formulation. The term "modified-release" encompasses "extended-release" and "delayed-release" formulations, as well as formulations having both extended-release and delayed-release characteristics. The administration of a modified-release formulation to a subject can be designed to alter one of many pharmacokinetic parameters of a pharmaceutically active compound in a subject by influencing its release rate. Examples of such pharmacokinetic parameters include, but are not limited to, the maximum plasma concentration (Cmax), the time to achieve a maximum plasma concentration following administration of the formulation (Tmax), the area under the plasma concentration-time curve (AUC), peak:trough fluctuation ratio (also called the peak:trough plasma ratio, or Fluctuation Index (FI)), the apparent elimination half-life (t_{1/2}), the apparent rate of elimination (Kₑₗᵢₘ), the apparent clearance calculated as dose/AUC (Cl), and the apparent volume of distribution (V_{d}).

An "extended-release" formulation can extend the time during which a given plasma concentration of a pharmaceutically active compound is maintained or the time during which an influence or effect of a therapeutically effective dose of a pharmaceutically active compound is observed in a subject, relative to conventional formulations. Such formulations are referred to herein as "extended-release formulations."

A "delayed-release" formulation can be designed to delay the release of the pharmaceutically active compound for a specified period. Such formulations are referred to herein as "delayed-release" or "delayed-onset" formulations or dosage forms.

As used herein, the term "immediate-release formulation," is meant to describe those formulations comprising N-2,3,3-tetramethylbicyclo-[2-1.1]heptan-2-amine in which more than about 50% of N-2,3,3-tetramethylbicyclo-[2.1.1]heptan-2-amine is released from the dosage form in less than about 2 hours. Such formulations are also referred to herein as "conventional formulations."

The formulations of the present invention are meant to encompass those that contain all possible combinations of components that exhibit modified-release and a combination of modified-release and immediate-release properties. For example, a formulation of the invention can contain components that exhibit both extended-release and immediate-release properties, or both delayed-release and immediate-release properties, or both extended-release and delayed-release properties, or a combination of all three properties. For example, a formulation including both immediate-release and extended-release components can be combined in a capsule, which is then coated with an enteric coat to provide a delayed-release effect.

The present inventive formulations are suitable for, but not limited to, oral, intra-nasal, buccal, sublingual, and transdermal administration, any of which can take the form of a modified-release or a combined modified-release and immediate-release formulation.

As used herein, the term ''intra-nasal" administration is meant to encompass those modes of administering a compound to a subject by means of absorption through the mucous membranes of the nasal cavity, or any administration that is made through the nasal cavity.

As used herein, the term "oral" is meant to encompass those modes of administering a compound to a subject via the mouth. The term oral encompasses the terms "buccal administration" and "sublingual administration," which are meant to encompass those modes of administering a compound to a subject by means of absorption through the mucous membranes of the oral cavity, or any administration that is made where the drug is absorbed from the mouth.

As used herein, the term "transdermal administration" is meant to encompass those modes of administering a compound to a subject by means of absorption through the skin. The term "transdermal formulation" is meant to encompass those pharmaceutical formulations, devices, and modes of administration, that are suitable for the transdermal administration of a compound in a subject. Such formulations can include pharmaceutically inert carriers or agents that are suitable, in addition to a pharmaceutically active compound.

As used herein, the term "pharmaceutically acceptable excipient" includes compounds that are compatible with the other ingredients in a pharmaceutical formulation and not injurious to the subject when administered in therapeutically acceptable amounts.

As used herein, the term "pharmaceutically acceptable salt" includes salts that are physiologically tolerated by a subject. Such salts can be prepared from an inorganic and/or organic acid. Examples of suitable inorganic acids include, but are not limited to, hydrochloric, hydrobromic, hydroiodic, nitric, sulfuric, and phosphoric acid. Organic acids can be aliphatic, aromatic, carboxylic, and/or sulfonic acids. Suitable organic acids include, but are not limited to, formic, acetic, propionic, succinic, camphorsulfonic, citric, fumaric, gluconic, lactic, malic, mucic, tartaric, paratoluenesulfonic, glycolic, glucuronic, maleic, furoic, glutamic, benzoic, anthranilic, salicylic, phenylacetic, mandelic, pamoic, methanesulfonic, ethanesulfonic, pantothenic, benzenesulfonic (besylate), stearic, sulfanilic, alginic, galacturonic, and the like.

The phrase "gastrointestinal motility reducing amount" of N-2,3,3-tetramethylbicyclo-[2.1.1]heptan-2-amine, as used herein, refers to the amount of N-2,3,3-tetramethylbicydo-[2.1.1]heptan-2-amine, or a pharmaceutically acceptable salt thereof, which alone or in combination with other drugs, provides any decrease in gastrointestinal motility. Thus, the N-2,3,3-tetramethylbicyclo-[2.1.1]heptan-2-amine can provide a therapeutic benefit in the reduction, prevention, and/or management of conditions or diseases that cause an increase in gastrointestinal motility. The term "treatment" as used herein refers to any reduction of gastrointestinal motility observed upon use of a method or formulation according to the present invention. The term "therapeutically effective amount,* as used herein, refers to the amount of N-2,3,3-tetramethylbicyclo-[2.1.1]heptan-2-amine, or a pharmaceutically acceptable salt thereof, which alone or in combination with other drugs, that is sufficient to reduce at least one symptom of conditions or diseases that cause an increase in gastrointestinal motility, which include, but are not limited to, abdominal discomfort and audible bowel noises, cramping and abdominal pain, an urgency to defecate, the passage of loose stools covered with mucus, and diarrhea.

The term "side effects," as used herein refers to physiological effects observed in a subject following administration of N-2,3,3-tetramethylbicyclo-[2.1.1]heptan-2-amine, other than a decrease in gastrointestinal motility, which may or may not result from an effect on the parasympathetic system. For example, such effects include, but are not limited to, effects on a subject's heart rate, blood pressure, vision, and bladder function. Side effects are often undesirable.

As used herein, the term "first administration" refers to the initial administration of a formulation of the invention to a subject. Alternatively, it refers to a single administration of a formulation of the invention to a subject.

In accordance with the invention, N-2,3,3-tetramethylbicyclo-[2.1.1]heptan-2-amine, or a pharmaceutically acceptable salt thereof, can be formulated and/or dosed in a manner that maximizes its gastrointestinal motility reducing effects while minimizing at least one side effect, such as effects on a subject's blood pressure, heart rate, vision, and bladder function.

In some aspects of the invention, a maximum plasma concentration of N-2,3,3-tetramethylbicyclo-[2.1.1]heptan-2-amine is achieved at about 3.5 hours, or later, following a first administration of a formulation of the invention. The maximum plasma concentration of N-2,3,3-tetramethylbicyclo-[2.1.1]heptan-2-amine can be achieved at about 3.5,4, 5, 6, 7, 8, 9, 10, 12, 14,18, or 24 hours, or later, or anytime in between, following a first administration according to the present invention.

Furthermore, the inventive formulations suitable for oral, intra-nasal, buccal or sublingual administration provide an in vitro release profile in which release of N-2,3,3-tetramethylbicyclo-[2.1.1]heptan-2-amine is minimal before about 2 hours and release is complete at about 24 hours. Alternatively, the formulations can provide equal-to or less than about 50% release at about 2 hours, less than or equal to about 70% release at about 4 hours, greater than or equal to about 50% release at about 8 hours, greater than or equal to about 65% release at about 12 hours, and greater than or equal to about 80% release at about 24 hours. Alternatively, less than or equal to about 40% is released at about 2 hours, less than or equal to about 65% is released at about 4 hours, greater than or equal to about 60% is released at about 8 hours, greater than or equal to about 70% is released at about 12 hours, and greater than or equal to about 80% is released at about 24 hours. Alternatively, less than or equal to about 30% is released at about 2 hours, about 20% to about 60% is released at about 4 hours, greater than or equal to about 70% is released at about 8 hours, greater than or equal to about 75% is released at about 12 hours, and greater than or equal to about 80% is released at about 24 hours.

Furthermore, the inventive formulations suitable for transdermal administration provide an in vitro release profile in which release is minimal before about 2 hours and release is complete at about 24 or more hours. Alternatively, the formulations can provide less than or equal to about 40% release at about 2 hours, about 10% to about 70% release at about 4 hours, about 20% to about 80% release at about 8 hours, greater than or equal to about 40% release at about 12 hours, and greater than of equal to about 70% release at about 24 hours. Alternatively, less than or equal to about 30% is released at about 2 hours, about 15% to about 60% is released at about 4 hours, about 30% to about 70% is released at about 8 hours, greater than or equal to about 50% is released at about 12 hours, and greater than or equal to about 75% is released at about 24 hours. Alternatively, less than or equal to about 25% is released at about 2 hours, about 20% to about 50% is released at about 4 hours, about 40% to about 70% is released at about 8 hours, greater than or equal to about 55% is released at about 12 hours, and greater than or equal to about 80% is released at about 24 hours.

The present invention also provides the use of a gastrointestinal motility reducing amount of N-2,3,3-tetramethylbicyclo-[2.1.1]heptan-2-amine, or a pharmaceutically acceptable salt thereof, in combination with at least one additional pharmaceutically active compound, for the manufacture of a medicament for reducing gastrointestinal motility in a subject suffering from an abnormal increase in gastrointestinal motility. Examples of other pharmaceutically active compounds that can be used in combination with N-2,3,3-tetramethylbicyclo-[2.1.1]heptan-2-amine include, but are not limited to, other ganglionic blockers and/or nicotinic-receptor antagonists (such as hexamethonium, trimethaphan, chloroisondamine, erysodine, β-dihydroerythrodine, amantidine, perpidine, succinylcholine, decamethonium, tubocurarine (including isomers thereof such as d-tubocurarine), atracurium, doxacurium, mivicurium, pancuronium, rocuronium, and vencuronium, for example), agents that alter gastrointestinal motility, antispasmodics, antimuscarinic agents (such as atropine and scopolamine), glycopyrrolate, hyscomine, opiates (such as loperamide, difenoxine, codeine, morphine, oxymorphone, oxycontin, dihydrocodeine, and fentanyl, for example), 5-HT receptor agonists, 5-HT antagonists (such as alosetron hydrochloride, for example), calcium channel blockers (such as verapamil, including its intestinal-selective isomer), beta blockers including beta blockers having effects on gastrointestinal function through neurogenic activity, agents used to treat various gastrointestinal symptoms and diseases including those that alter fluid transport across the gut or into or out of gastrointestinal cells, diuretics (such as amiloride and furosemide, for example), anti-diarrheals (such as bismuth and sandostatin, for example), H₂-antihistamines, proton pump inhibitors, antacids, anti-inflammatory agents, sulfasalazine, steroids (such as mineralocorticoids, corticosteroids, estrogens, prednisone, prednisolone, cortisol, cortisone, fluticasone, dexamethasone, and betamethasone, for example), 5-aminosalicylic acid, anti-infective agents (such as metronidazole, ciprofloxacin, and azathioprine, for example), immunomodulators (such as 6-mercaptopurine, cyclosporine, and methotrexate, for example), fish oil, remicade, heparin, and nicotine.

N-2,3,3-tetramethylbicyclo-[2.1.1]heptan-2-amine, or a pharmaceutically acceptable salt thereof, can be administered with one or more of such pharmaceutically active compounds. Combinations can be administered such that N-2,3,3-tetramethylbicyclo-[2.1.1]heptan-2-amine, or a pharmaceutically acceptable salt thereof, and the at least one other pharmaceutically active compound are contained in the same dosage form. Alternatively, the combinations can be administered such that N-2,3,3-tetramethyibicycio-[2.1.1]heptan-2-amine and the at least one additional pharmaceutically active compound are contained in separate dosage forms and are administered concomitantly or sequentially. Combinations of the above-listed pharmaceutically active compounds with racemic N-2,3,3-tetramethylbicyclo-[2.1.1]heptan-2-amine, enriched (R)-N-2,3,3-tetramethylbicyclo-[2.1.1]heptan-2-amine, enriched (S)-N-2,3,3-tetramethylbicyclo-[2.1.1]heptan-2-amine, substantially pure (R)-N-2,3,3-tetramethylbicyclo-[2.1.1]heptan-2-amine, or substantially pure (S)-N-2,3,3-tetramethylbicyclo-[2.1.1]heptan-2-amine or pharmaceutically acceptable salts thereof, are also specifically contemplated.

The present invention also provides the use of a gastrointestinal motility reducing amount of enriched (R)-N-2,3,3-tetramethylbicyclo-[2.1.1]heptan-2-amine, enriched (S)-N-2,3,3-tetramethylbicyclo-[2.1.1]heptan-2-amine, substantially pure (R)-N-2,3,3-tetramethylbicyclo-[2.1.1]heptan-2-amine, or substantially pure (S)-N-2,3,3-tetramethylbicyclo-[2.1.1]heptan-2-amine or pharmaceutically acceptable salts thereof, for the manufacture of a medicament for reducing gastrointestinal motility in a subject suffering from an abnormal increase in gastrointestinal motility. Such formulations can comprise enriched (R)-N-2,3,3-tetramethylbicyclo-[2.1.1]heptan-2-amine, enriched (S)-N-2,3,3-tetramethylbicyclo-[2.1.1]heptan-2-amine, substantially pure (R)-N-2,3,3-tetramethylbicyclo-[2.1.1]heptan-2-amine, or substantially pure (S)-N-2,3,3-tetramethylbicyclo-[2.1.1]heptan-2-amine, or pharmaceutically acceptable salts thereof, in which the (R)-stereoisomer or (S)-stereoisomer, respectively, comprises about 51% to about 100% or about 61% to about 100%, or about 71% to about 100%, or about 81% to about 100%, or about 91% to about 100%, or about 95% to about 100%, of the total amount of N-2,3,3-tetramethylbicyclo-[2.1.1]heptan-2-amine present.

In accordance with the invention, the maximum plasma concentration of enriched or substantially pure (R)-N-2,3,3-tetramethylbicyclo-[2.1.1]heptan-2-amine or enriched, or substantially pure (S)-N.2,3,3-tetramethylbicyclo- . [2.1.1]heptan-2-amine can be achieved at about 3.5, 4, 5, 6, 7, 8, 9, 10, 12, 14, 18, or 24 hours, of later, or any time in between, following a first administration of enriched or substantially pure (R)-N-2,3,3-tetramethylbicyclo-[2.1.1]heptan-2-amine, or enriched or substantially pure (S)-N-2,3,3-tetramethytbicycio-[2.1.1]heptan-2-amine, or pharmaceutically acceptable salts thereof, respectively, according to the present invention.

The N-2,3,3-tetramethylbicyclo-[2.1.1] heptan-2-amine used in accordance with the present invention can be obtained by any method. Examples of such methods are described in U.S. Patent Nos. 2,831,027, 2,885,428, and 5,986,142. Modifications of the protocols described in these patents, as well as other routes of synthesis, are well known to those of ordinary skill in the art and can be employed in accordance with the present invention.

Mixtures of any and all possible stereoisomers of N-2,3,3-tetramethylbicyclo-[2.1.1]heptan-2-amine, or pharmaceutically acceptable salts thereof, can be obtained by any method suitable for that purpose. For example, a racemic mixture of *exo*-(R)-N-2,3,3-tetramethylbicyclo-[2.1.1]heptan-2-amine and *exo*-(S)-N-2,3,3-tetramethylbicyclo-[2,1,1]heptan-2-amine, or pharmaceutically acceptable salts thereof, can be obtained by the methods disclosed in U.S. Patent Nos. 2,831,027, 2,885,428, and 5,986,142. Enriched (R)-N-2,3,3-tetramethylbicyclo-[2.1.1]heptan-2-amine, enriched (S)-N-2,3,3-tetramethylbicyclo-[2.1.1]heptan-2-amine, substantially pure (R)-N-2,3,3-tetramethylbicyclo-[2.1.1]heptan-2-amine, or substantially pure (S)-N-2,3,3-tetramethylbicyclo-[2.1.1]heptan-2-amine or pharmaceutically acceptable salts thereof, can be obtained by the methods disclosed in U.S. Patent No. 5,039,801 or U.S. Pat. App. Publication 20020016371 A1, for example.

The pharmaceutically acceptable formulations described herein can be provided in the form of a pharmaceutical formulation for use according to the present invention. Such formulations optionally include one or more pharmaceutically acceptable excipients. Examples of suitable excipients are known to those of skill in the art and are described, for example, in the Handbook of Pharmaceutical Excipients (Kibbe (ed.), 3rd Edition (2000), American Pharmaceutical Association, Washington, D.C.), and Remington: The Science and Practice of Pharmacy (Gennaro (ed.), 20th edition (2000), Mack Publishing, Inc., Easton, PA) (hereinafter referred to as *"Remington"*)*.*

Suitable excipients include; but are not limited to, starches, sugars, microcrystalline cellulose, diluents, granulating agents, lubricants, binders, disintegrating agents, wetting agents, emulsifiers, coloring agents, release agents, coating agents, sweetening agents, flavoring agents, perfuming agents, preservatives, plasticizers, gelling agents, thickeners, hardeners, setting agents, suspending agents, surfactants, humectants, carriers, stabilizers, antioxidants, and combinations thereof.

The pharmaceutical formulations of the invention can be provided in dosage forms that are suitable for administration to a subject by a desired route. A number of suitable dosage forms are described below, but this description is not meant to include all possible choices. One of skill in the art is familiar with the various dosage forms that are suitable for use in the present invention, as described, for example, in *Remington.* The most suitable route in any given case will depend on the nature and severity of the condition being prevented, treated, and/or managed. The pharmaceutical formulations of this invention can be formulated for administration orally, nasally, buccally, sublingually, rectally, intravaginally, parenterally, intracisternally, topically, and transdermally.

Formulations suitable for oral administration include, but are not limited to, capsules, cachets, pills, tablets, lozenges (using a flavored base, usually sucrose and acacia or tragacanth), powders, granules, solutions, suspensions in an aqueous or non-aqueous liquid, oil-in-water or water-in-oil liquid emulsions, elixirs, syrups, pastilles (using an inert base, such as gelatin and glycerin, or sucrose and acacia), mouth washes, pastes, and the like, each containing a predetermined amount of N-2,3,3-tetramethylbicyclo-[2.1.1]heptan-2-amine, or a pharmaceutically acceptable salt thereof, to provide a therapeutic amount of the drug in one or more doses.

N-2,3,3-Tetramethylbicyclo-[2.1.1]heptan-2-amine, or a pharmaceutically acceptable salt thereof, can be mixed with pharmaceutically acceptable excipients in the preparation of dosage forms for oral administration (capsules, tablets, pills, powders, granules and the like). Suitable excipients include, but are not limited to, carriers, such as sodium citrate or dicalcium phosphate; fillers or extenders, such as starches, lactose, sucrose, glucose, mannitol, or silicic acid; binders, such as hydroxymethyl-cellulose, alginates, gelatin, polyvinylpyrrolidone, sucrose or acacia; humectants; such as glycerol; disintegrating agents, such as agar, calcium carbonate, potato or tapioca starch, alginic acid, certain silicates, or sodium carbonate; solution retarding agents, such as paraffin; absorption accelerators, such as quaternary ammonium compounds; wetting agents, such as cetyl alcohol or glycerol monostearate; absorbents, such as kaolin and bentonite clay; lubricants, such as talc, calcium stearate, magnesium stearate, solid polyethylene glycols, and sodium lauryl sulfate; coloring agents; buffering agents; dispersing agents; preservatives; and diluents. The aforementioned excipients are given as examples only and are not meant to include all possible choices. Solid formulations can also be employed as fillers in soft and hard-filled gelatin capsules using excipients such as lactose or milk sugars, high molecular weight polyethylene glycols, and the like. Any of these dosage forms can optionally be scored or prepared with coatings and shells, such as enteric coatings and coatings for modifying the rate of release, examples of which are well known in the pharmaceutical-formulating art.

Such coatings can comprise sodium carboxymethylcellulose, cellulose acetate, cellulose acetate phthalate, ethylcellulose, gelatin, pharmaceutical glaze, hydroxypropyl cellulose, hydroxypropyl methylcellulose, hydroxypropyl methylcellulose phthalate, methacrylic acid copolymer, methylcellulose, polyethylene glycol, polyvinyl acetate phthalate, shellac, sucrose, titanium dioxide, wax, or zein. In one embodiment, the coating material comprises hydroxypropyl methylcellulose. The coating material can further comprise anti-adhesives, such as talc; plasticizers (depending on the type of coating material selected), such as castor oil, diacetylated monoglycerides, dibutyl sebacate, diethyl phthalate, glycerin, polyethylene glycol, propylene glycol, triacetin, triethyl citrate; opacifiers, such as titanium dioxide; and/or coloring agents and/or pigments. The coating process can be carried out by any suitable means, for example, by using a perforated pan system such as the GLATT^{™}, ACCELACOTA^{™}, and/or HICOATER^{™} apparatuses.

The formulations of the present invention can exist as a multiparticulate formulation. The term "multiparticulate" as used herein means a plurality of discrete or aggregated particles, beads, pellets, granules; tablets, or mixture thereof without regard to their size, shape, or morphology.

Tablets can be formed by any suitable process, examples of which are known to those of ordinary skill in the art. For example, the ingredients can be dry-granulated or wet-granulated by mixing in a suitable apparatus before being formed into tablets. Granules of the ingredients to be formed into tablets can also be prepared using suitable spray/fluidization or extrusion/spheronisation techniques.

The tablets can be formulated with suitable excipients to act as a fast dissolving and/or fast melting tablet in the oral cavity. Also, the tablet can be in the form of a chewable or effervescent dosage form. With effervescent dosage forms, the tablet can be added to a suitable liquid that causes it to disintegrate, dissolve, and/or disperse.

Tablets can be designed to have an appropriate hardness and friability to facilitate manufacture on an industrial scale using equipment to produce tablets at high speed. Also, the tablets can be packed or filled in any kind of container. It should be noted that the hardness of tablets, among other properties, can be influenced by the shape of the tablets. Different shapes of tablets can be used according to the present invention. Tablets can be circular, oblate, oblong, or any other shape. The shape of the tablets can also influence the disintegration rate.

Any of the inventive formulations can be encapsulated in soft and hard gelatin capsules, which can also include any of the excipients described above. For example, the encapsulated dosage form can include fillers, such as lactose and microcrystalline glidants, such as colloidal silicon dioxide and talc; lubricants, such as magnesium stearate; and disintegrating agents, such as starch (*e.g.*, maize starch). Using capsule filling equipment, the ingredients to be encapsulated can be milled together, sieved, mixed, packed together, and then delivered into a capsule...Lubricants can be present in an amount of from about 0.5% (w/w) to about 2.0% (w/w). In one embodiment, the lubricant is about 1.25% (w/w) of the content of the capsule.

The formulations of the invention, which comprise N-2,3,3-tetramethylbicyclo-[2.1.1]heptan-2-amine, or a pharmaceutically acceptable salt thereof, can also be formulated into a liquid dosage form for oral administration. Suitable formulations can include emulsions, microemulsions, solutions, suspensions, syrups, and elixirs. N-2,3,3-tetramethylbicyclo-[2.1.1]heptan-2-amine can be formulated as an ion-exchange resin complex, a microencapsulated particle, a liposome particle, or a polymer coated particle or granule. These formulations optionally include diluents commonly used in the art, such as, for example, water or other solvents, solubilizing agents and emulsifiers. Emulsifiers include, but are not limited to, ethyl alcohol, isopropyl alcohol, ethyl carbonate, ethyl acetate, benzyl alcohol, benzyl benzoate, propylene glycol, 1,3-butylene glycol, oils, glycerol, tetrahydrofurfuryl alcohol, polyethylene glycols, fatty acid esters of sorbitan, and mixtures thereof. In addition, the inventive formulations can include adjuvants such as wetting agents, emulsifying and suspending agents, sweetening, flavoring, coloring, perfuming, and preservative agents. Suitable suspension agents include, but are not limited to, ethoxylated isostearyl alcohols, polyoxyethylene sorbitol and sorbitan esters, microcrystalline cellulose, aluminum metahydroxide, bentonite, agar-agar and tragacanth, and mixtures thereof. The liquid formulations can be delivered as-is, or can be provided in hard or soft capsules, for example.

The amount of suspending agent present will vary according to the particular suspending agent used, and the presence or absence of other ingredients that have an ability to act as a suspending agent or contribute significantly to the viscosity of the formulation. The suspension can also contain ingredients that improve its taste, for example sweeteners; bitter-taste maskers, such as sodium chloride; taste-masking flavors, such as contramarum; flavor enhancers, such as monosodium glutamate; and flavoring agents. Examples of sweeteners include bulk sweeteners, such as sucrose, hydrogenated glucose syrup, the sugar alcohols sorbitol and xylitol; and sweetening agents such as sodium cyclamate, sodium saccharin, aspartame, and ammonium glycyrrhizinate. The liquid formulations can further comprise one or more buffering agents, as needed, to maintain a desired pH.

The liquid formulations of the present invention can also be filled into soft gelatin capsules. The liquid can include a solution, suspension, emulsion, microemulsion, precipitate, or any other desired liquid media carrying the pharmaceutically active compound. The liquid can be designed to improve the solubility of the pharmaceutically active compound upon release, or can be designed to form a drug-containing emulsion or dispersed phase upon release. Examples of such techniques are well known in the art. Soft gelatin capsules can be coated, as desired, with a functional coating. Such functional coatings generally serve the purpose of delaying the release of the drug for a predetermined period. For example, such coatings can allow the dosage form to pass through the stomach without being subjected to stomach acid or digestive juices. Thus, such coatings can dissolve or erode upon reaching a desired point in the gastrointestinal tract, such as the upper intestine.

The formulations of the present invention can also be provided in a form suitable for intra-nasal administration. The nasal delivery of therapeutic agents is known in the art. See, for example, U.S. Pat, Nos. 4,428,883; 4,284,648, 4,394,390, and 4,77810. The formulations of the invention suitable for intra-nasal administration comprise N-2,3,3-tetramethylbicyclo-[2.1.1]heptan-2-amine, or a pharmaceutically acceptable salt thereof, and are in any form suitable for intra-nasal administration, including, but not limited to, gels, sprays and solutions which can be administered in the form of drops.

The formulations suitable for intra-nasal administration can be provided as isotonic aqueous solutions, suspensions, or viscous formulations, which can be buffered to a selected pH. The formulations can be in the form of gels, lotions, ointments, creams and the like and will typically contain a sufficient amount of a thickening agent so that the viscosity is from about 2500 to about 6500 cps, although more viscous formulations, even up to about 10,000 cps can be employed.

The concentration of N-2,3,3-tetramethylbicyclo-[2.1.1]heptan-2-amine in the formulations for intra-nasal administration can vary according to factors such as the condition being treated, the age, and the weight (or size) of the subject. The formulations of the invention can contain N-2,3,3-tetramethylbicyclo-[2.1.1]heptan-2-amine, or a pharmaceutically acceptable salt thereof, in a concentration of from about 1 mg/mL to about 2000 mg/mL. The volume of a dosage unit can be from about 0.05 mL to about 0.3 mL.

The desired isotonicity of the formulation can be achieved using sodium chloride, or other pharmaceutically acceptable agents such as dextrose, boric acid, sodium tartrate, propylene glycol, or other inorganic or organic solutes.

The viscosity of the formulations can be maintained at the desired level using a pharmaceutically acceptable thickening agent. Suitable thickening agents include, but are not limited to, methyl cellulose, xanthan gum, carboxymethyl cellulose, hydroxypropyl cellulose, and carbomer.

Formulations suitable for intra-nasal administration can also contain a pharmaceutically acceptable humectant to inhibit drying of the mucous membrane and prevent irritation. Pharmaceutically acceptable humectants that can be used include, but are not limited to, sorbitol propylene glycol or glycerol. The concentration of the selected humectant will vary with the selected agent.

Enhanced absorption across the nasal membrane can be accomplished by employing a pharmaceutically acceptable surfactant. Pharmaceutically acceptable surfactants that can be used include, but are not limited to, polyoxyethylene derivatives of fatty acid partial esters of sorbitol anhydrides such as Tween 80, Polyoxyl 40 Stearate, Polyoxyethylene 50 Stearate and Octoxynol. These surfactants can be used in a range of from about 1% to about 10% based on the total weight of the formulation.

The intra-nasal formulations can also include a pharmaceutically acceptable preservative. Preservatives that can be used include, but are not limited to, benzyl alcohol, parabens, thimerosal, chlorobutanol, and benzalkonium chloride. These preservatives can be used in an amount ranging from about 0.02% to about 2%, based on the total weight of the formulation.

For buccal or sublingual administration, the formulations of the invention can be provided in the form of a tablet, patch, troche, or in free form, such as a gel, ointment, cream, or gum. Examples of suitable buccal or sublingual formulations and devices are disclosed, for example, in U.S. Patent Nos. 5,863,555, 5,849,322, 5,766,620, 5,516,523, 5,346,701, 4,983,395, and 4,849,224. Such formulations and devices can use a suitable adhesive to maintain the device in contact with the buccal mucosa. Examples of suitable adhesives are found, for example, in U.S. Pat. Nos. 3,972,995, 4,259,314, 4,680,323; 4,740,365, 4,573,996, 4,292,299, 4,715,369, 4,876,092, 4,855,142, 4,250,163, 4,226,848, and 4,948,580. Typically, the adhesive comprises a matrix of a hydrophilic, e.g., water soluble or swellable, polymer or mixture of polymers that can adhere to a wet, mucous surface. These adhesives can be formulated as ointments, thin films, tablets, troches, and other forms.

For rectal or vaginal administration, the inventive formulations can be provided as a suppository. Suppositories can comprise one or more nonirritating excipients such as, for example, polyethylene glycol, a suppository wax, or a salicylate. Such excipients can be selected on the basis of desirable physical properties. For example, a compound that is solid at room temperature but liquid at body temperature will melt in the rectum or vaginal cavity and release the active compound. The formulation can alternatively be provided as an enema for rectal delivery. Formulations suitable for vaginal administration also include pessaries, tampons, creams, gels, pastes, foams, or spray formulations containing such carriers, examples of which are known in the art.

Formulations suitable for topical or transdermal administration include, but are not limited to, powders, sprays, ointments, pastes, creams, lotions, gels, solutions, patches, and inhalants. Such formulations can contain excipients such as animal and vegetable fats, oils, waxes, paraffins, starch, tragacanth, cellulose derivatives, polyethylene glycols, silicones, bentonites, silicic acid, talc, zinc oxide, or mixtures thereof. Powders and sprays can also contain excipients such as lactose, talc, silicic acid, aluminum hydroxide, calcium silicates, and polyamide powder. Additionally, sprays can contain propellants, such as chlorofluoro-hydrocarbons and volatile unsubstituted hydrocarbons, such as butane and/or propane.

The systemic delivery of pharmaceutically active compounds via transdermal administration has the advantages of the accessibility of the skin as well as subject acceptability and compliance. In general, inventive transdermal delivery devices can be divided into categories, including, but not limited to, membrane-modulated, adhesive diffusion-controlled, matrix-dispersion-type, and microreservoir systems. See, *Remington,* Chapter 47, pp. 903-929.

For membrane-modulated systems, the drug reservoir is generally encapsulated in a shallow compartment molded from a drug-impermeable backing and a rate-controlling polymeric membrane. N-2,3,3-tetramethylbicyclo-[2.1.1]heptan-2-amine is released through the rate-controlling membrane, which can be microporous or nonporous. On the external surface of the membrane, a layer of drug-compatible, hypoallergenic, adhesive polymer can be applied to achieve contact of the delivery device with the subject's skin. Examples of the drug-compatible, hypoallergenic, adhesive polymer include, but are not limited to, silicone and polyacrylate adhesives. The rate of drug release can be altered by varying the polymer composition, permeability coefficient, or thickness of the rate-limiting membrane and adhesive.

In adhesive diffusion-controlled transdermal systems, the drug reservoir is generally formulated by directly dispersing the drug in an adhesive polymer matrix and spreading the dispersion onto a flat sheet of drug-impermeable backing to form a thin drug-reservoir layer. On top of this layer are placed further layers of non-drug containing adhesive polymers of constant thickness. The adhesive matrix can be prepared by mixing a solution of adhesive polymer, which can be purchased commercially, or by dissolving an adhesive solid in a suitable solvent, with a solution of N-2,3,3-tetramethylbicyclo-[2.1.1]heptan-2-amine dissolved or evenly dispersed, in enhancers if desired. The mixture can be poured into a mold or cast alone or on a desired backing material. The casting can be left for the solvent to evaporate at room temperature or in an oven at a slightly elevated temperature. After solvent evaporation, the adhesive matrix takes the form of an adhesive polymer film, which can have a thickness in the range of about from 50 to 100 µm.

Matrix dispersion-type transdermal systems generally include drug reservoirs that are formed by dispersing a drug in a hydrophobic or lipophilic polymer and then molding it into a disk with a defined surface area and controlled thickness. Optionally, the drug may be homogenously dispersed. The disk can be glued onto an occlusive baseplate in a compartment prepared from a drug-impermeable backing. The adhesive polymer can be spread along the circumference of the disk to form a rim, which can then be applied to a subject's skin.

In microreservoir systems, the drug reservoir can be prepared by suspending the drug particles in an aqueous solution of water-soluble polymer and then dispersing it in a lipophilic polymer, for example, by high-shear mechanical force to form unleachable, microscopic spheres of drug. Optionally, the drug may be homogenously dispersed. The spheres are effective to release entrapped drug at a rate sufficient to achieve the desired skin permeation rate. Such particles can include a hydrophilic polymer chosen, for example, from polyvinyl alcohol, polyvinylpyrrilodone, polyacrylic acid, and celluloses. The particles can be liposomes. The dispersion is then stabilized by cross-linking the polymer in situ, producing a disk containing drug with a constant surface area and fixed thickness. The disk can then be positioned at the center of a transdermal system surrounded by an adhesive rim.

In transdermal formulations according to the invention, pharmaceutically active compounds can be present in any layers that comprise the transdermal delivery device. The amount of pharmaceutically active compounds present in each layer can be varied according to the desired rate of release for each. For example, an amount of N-2,3,3-tetramethylbicyclo-[2.1.1]heptan-2-amine loaded into the adhesive matrix can be varied by varying its concentration in the casting mixture and the thickness of the adhesive matrix. The amount of N-2,3,3-tetramethylbicyclo-[2.1.1]heptan-2-amine in the adhesive matrix of a given patch area should be sufficient to provide a gastrointestinal motility reduction effect over the range of about 4 hours to about 7 days, or over the range of about 4 hours to about 72 hours, or over the range of about 4 to about 48 hours, or over the range of about 4 to about 24 hours, or any number of hours in between.

The transdermal devices according to the present invention can include N-2,3,3-tetramethylbicyclo-[2.1.1]heptan-2-amine formulated and incorporated into the transdermal system in a microencapsulated or liposomal form. These forms can improve processing, stability, tolerability, or delivery characteristics of the system.

The transdermal devices according to the present invention can also include an enhancer effective to increase the skin permeation rate of N-2,3,3-tetramethylbicyclo-[2.1.1]heptan-2-amine. Enhancers that can be advantageously used to enhance the transdermal administration of N-2,3,3-tetramethylbicyclo-[2.1.1]heptan-2-amine include, but are not limited to, fatty acids, fatty acid esters, and fatty alcohols. Such compounds generally are hydrophobic or have limited water solubility, and the compounds can have a molecular weight of from about 150 to about 300 Daltons. Fatty alcohols include, but are not limited to, stearyl alcohol and oleyl alcohol. Fatty acids include, but are not limited to, oleic acid, lauric acid, myristic acid, palmitic acid, stearic acid, linoleic acid, caprylic acid, monoglycerides, diglycerides, acylcholines, caprylic acids, acylcarnitines, sodium caprate, and palmitoleic acid. Fatty acid esters containing 10, 11, 12 or more carbons can also be used. Examples of fatty acid esters include, but are not limited to, isopropyl myristate and methyl and ethyl esters of oleic and lauric acid.

Ionic enhancers can also be used. Ionic enhancers that can be used include, but are not limited to, sodium lauryl sulfate, sodium laurate, polyoxyethylene20-cetyether, laureth-9, sodium dodecylsulfate, and dioctyl sodium sulfosuccinate.

Bile salts can also be used. Bile salts that can be used include, but are not limited to, sodium glycocholate, sodium deoxycholate, sodium taurocholate, sodium taurodihydrofusidate, and sodium glycodihydrofusidate.

Chelating agents can also be used as enhancers Examples of chelating agents that can be used include, but are not limited to, EDTA, citric acid, and salicylates.

Another group of enhancers includes low molecular weight alcohols. Such alcohols can have a molecular weight of less than about 200 Daltons, or less than about 150 Daltons, or less than 100 Daltons. They can also be hydrophilic, generally having greater than 2 wt%, 5 wt%, or 10 wt% solubility in water at room temperature. Examples of such alcohols include, but are not limited to, methanol, ethanol, propanol, isopropanol, butanol, benzyl alcohol, glycerin, polyethylene glycol, propanediol, and propylene glycol.

Sulfoxides can also be used as enhancers. Examples of sulfoxides include, but are not limited to, dimethyl sulfoxide and decmethyl sulfoxide.

Other enhancers that can be used include, but are not limited to, urea and its derivatives, unsaturated cyclic ureas, 1-dodecylazacycloheptan-2-one, cyclodextrin, enamine derivatives, terpenes, liposomes, acyl carnitines, cholines, peptides (including polyarginine sequences or arginine rich sequences), peptidomimetics, diethyl hexyl phthalate, octyldodecyl myristate, isostearyl isostearate, caprylic/capric triglyceride, glyceryl oleate, and various oils (such as wintergreen or eucalyptol).

Other examples of enhancers suitable for use in the present invention are provided by Santus, G. C. et al., Journal of Controlled Release, 25:1-20 (1993), and *Remington.*

Transdermal formulations according to the invention can include at least one pharmaceutically active compound in addition to N-2,3,3-tetramethylbicyclo-[2.1.1]heptan-2-amine. Among the at least one additional pharmaceutically active compounds that can be used in the present invention are, for example, other ganglionic blockers and/or nicotinic-receptor antagonists (such as hexamethonium, trimethaphan, chloroisondamine, erysodine, β-dihydroerythrodine, amantidine, perpidine, succinylcholine, decamethonium, tubocurarine, (including isomers thereof such as d-tubocurarine), atracurium, doxacurium, mivicurium, pancuronium, rocuronium, and vencuronium, for example), agents that alter gastrointestinal motility, antispasmodics, antimuscarinic agents, glycopyrrolate, atropine, hyscomine, scopolamine, opiates (such as loperamide, difenoxine, codeine, morphine, oxymorphone, oxycontin, dihydrocodeine, and fentanyl, for example), 5-HT receptor agonists 5-HT antagonists (such as alosetron hydrochloride, for example) calcium channel blockers (such as verapamil, including its intestinal selective isomers, for example), beta blockers, beta blockers having effects on gastrointestinal function through neurogenic activity, agents used to treat various gastrointestinal symptoms and diseases including those that alter fluid transport across the gut or into or out of gastrointestinal cells, diuretics (such as amiloride and furosemide, for example) anti-diarrheals (such as bismuth and sandostatin, for example) H₂-antihistamines, proton pump inhibitors, antacids, anti-inflammatory agents, sulfasalazine, steroids (such as mineralocorticoids, corticosteroids, estrogens, prednisone, prednisolone, cortisol, cortisone, fluticasone, dexamethasone, and betamethasone, for example), 5-aminosalicylic acid, anti-infective agents (such as metronidazole, ciprofloxacin, and azathioprine, for example), immunomodulators (such as 6-mercaptopurine, cyclosporine, and methotrexate, for example), fish oil, remicade, heparin, and nicotine.

The adhesive used in an adhesive matrix-type transdermal patch can be selected from any adhesive acceptable for use in pharmaceutical patches. For example, an adhesive can be based on polyisobutylene, acrylics, or silicone. The adhesive selected can depend in part on the enhancer or enhancers chosen, and the amount of drug and enhancer loaded into the matrix. The adhesive should retain its adhesive properties in the presence of these additives, and provide tack for good instantaneous adhesion to the skin, good adhesion throughout the treatment period, and clean removal from the skin after treatment. Some suitable adhesives include those available from Avery Chemical Corp and from National Starch and Chemical Company.

Additionally, the transdermal patch of the invention can be used in combination with an energy-assisted device to enhance the delivery of N-2,3,3-tetramethylbicyclo-[2.1.1]heptan-2-amine. Examples of such energy assisted devices include, but are not limited to, iontophoretic, solar, and thermal devices.

In an iontophoresis drug delivery device, a battery can be connected to two electrodes in the device and the electrodes placed on the skin. The drug is placed in contact with one electrode (for example, a positive drug can be placed in contact with the positive electrode) and when a current of low voltage is applied across the electrodes, the drug will migrate through the skin toward the opposite electrode, thereby entering the body. The amount of drug delivered can be a function of the applied current and the treatment time, and these parameters are known to those of skill in the art. Iontophoresis and iontophoretic devices are discussed, for example, by Ranade et al, DRUG DELIVERY SYSTEMS, CRC Press, Chapter 6, (1996); Tyle, Pharmaceutical Res., 3:318 (1986); and Banga et al., J. Controlled Release, 7:1-24 (1988).

The release profiles and skin permeation rates of the transdermal formulations of the present invention can be determined using an in vitro diffusion test according to methods adapted from Franz, J. Invest. Dermatol. 64:194-195 (1975) and GB-A-2-098 865.

For parenteral administration, such as administration by injection (including, but not limited to, subcutaneous, bolus injection, intramuscular, intraperitoneal, and intravenous), the pharmaceutical formulations can be formulated as isotonic suspensions, solutions, or emulsions, in oily or aqueous vehicles, and can contain formulatory agents such as suspending, stabilizing, or dispersing agents. Alternatively, the formulations can be provided in dry form such as a powder, crystalline, or freeze-dried solid, for reconstitution with sterile pyrogen-free water or isotonic saline before use. They can be presented, for example, in sterile ampoules or vials.

Examples of suitable aqueous and nonaqueous excipients include water, ethanol, polyols (such as glycerol, propylene glycol, polyethylene glycol, and the like), oils, injectable organic esters, and mixtures thereof. Proper fluidity can be maintained, for example, by the use of surfactants.

These formulations can also contain adjuvants such as preservatives, wetting agents, emulsifying agents, and dispersing agents. Prevention of the action of microorganisms can be achieved by the inclusion of various antibacterial and/or antifungal agents, for example, paraben, chlorobutanol, phenol sorbic acid, and the like. It also can be desirable to include isotonic agents, such as sugars, sodium chloride, and the like in the formulations. In addition, prolonged absorption of the injectable pharmaceutical form can be brought about by the inclusion of agents that delay absorption, such as aluminum monostearate and/or gelatin.

To prolong or extend the therapeutic effect of a drug, it can be desirable to slow the absorption of the drug from a subcutaneous or intramuscular injection. This can be accomplished by the use of a liquid suspension of crystalline or amorphous material having low solubility. Alternatively, modified release of injected forms can be achieved by encapsulation of N-2,3,3-tetramethylbicyclo-[2.1.1]heptan-2-amine in a biodegradable or biocompatible polymer that controls the rate of drug release. Alternatively, liposome formulations can be used. The rate of absorption of the drug then generally depends upon its rate of dissolution, which can depend upon crystal size and crystalline form. Alternatively, delayed absorption of a parenterally-administered form can be accomplished by dissolving or suspending the drug in an oil vehicle.

In addition to the inventive dosage forms described herein, the formulations of the present invention can be formulated into an oral dosage form that modifies the release of N-2,3,3-tetramethylbicyclo-[2.1.1]heptan-2-amine. Examples of modified-release formulations are known in the art and are, for example, described In U.S. Pat. Nos.: 3,845,770; 3,916,899; 3,536,809; 3,598,123; 4,008,719; 5,674,533; 5,059,595; 5,591,767; 5,120,548; 5,073,543; 5,639,476; 5,354,556; and 5,733,566. Advantages of modified-release formulations can include extended activity of the drug, reduced dosage frequency, and increased subject compliance.

A number of modified dosage forms suitable for use are described below. A more detailed discussion of such forms can also be found in, for example The Handbook of Pharmaceutical Controlled Release Technology, D. L. Wise (ed.), Marcel Decker, Inc., New York (2000); and also in Treatise on Controlled Drug Delivery: Fundamentals, Optimization, and Applications, A. Kydonieus (ed.), Marcel Decker, Inc., New York, (1992).

Examples of modified or extended-release formulations include but are not limited to, diffusion-controlled, matrix, osmotic, and ionic exchange systems. These can be in the form of single (monolithic) or multiunit dosage forms. With diffusion-controlled extended release dosage forms, the formulation containing the active substance of interest can be surrounded by a semi-permeable membrane. Semi-permeable membranes include those that are permeable to a greater or lesser extent to both water and solute. This membrane can include water-insoluble and/or water-soluble polymers, and can exhibit pH-dependent and/or pH-independent solubility characteristics. Polymers of these types are described in detail below. Generally, the characteristics of the polymeric membrane (e.g., the composition of the membrane) will determine the nature of release from the dosage form.

Matrix-Based Dosage Forms

Matrix-type systems comprise an active substance of interest, mixed with either water-soluble, e.g., hydrophilic polymers, or water-insoluble, e.g., hydrophobic polymers. Generally, the properties of the polymer used in a modified-release dosage form will affect the mechanism of release. For example, the release of the active ingredient from a dosage form containing a hydrophilic polymer can proceed via both surface diffusion and/or erosion. Mechanisms of release from pharmaceutical systems are well known to those skilled in the art. Matrix-type systems can also be monolithic or multiunit, and can be coated with Water-soluble and/or water-insoluble polymeric membranes, examples of which are described above.

Matrix formulations of the present invention can be prepared by using, for example, direct compression or wet granulation. A functional coating, as noted above, can then be applied in accordance with the invention. Additionally, a barrier or sealant coat can be applied over a matrix tablet core prior to application of a functional coating. The barrier or sealant coat can serve the purpose of separating an active ingredient from a functional coating, which can interact with the active ingredient, or it can prevent moisture from contacting the active ingredient. Details of barriers and sealants are provided below.

In a matrix-based dosage form in accordance with the present invention, N-2,3,3-tetramethylbicyclo-[2.1.1]heptan-2-amine and optional pharmaceutically acceptable excipient(s) are dispersed within a polymeric matrix, which typically comprises one or more water-soluble polymers and/or one or more water-insoluble polymers. The drug can be released from the dosage form by diffusion and/or erosion. Such matrix systems are described in detail by Wise and Kydonieus, *supra.*

Suitable water-soluble polymers include, but are not limited to, polyvinyl alcohol, polyvinylpyrrolidone, methylcellulose, hydroxypropylcellulose, hydroxypropylmethyl cellulose, or polyethylene glycol, and/or mixtures thereof.

Suitable water-insoluble polymers include, but are not limited to, ethylcellulose, cellulose acetate, cellulose propionate, cellulose acetate propionate, cellulose acetate butyrate, cellulose acetate phthalate, cellulose triacetate, poly (methyl methacrylate), poly (ethyl methacrylate), poly (butyl methacrylate), poly (isobutyl methacrylate), poly (hexyl methacrylate), poly (isodecyl methacrylate), poly (lauryl methacrylate), poly (phenyl methacrylate), poly (methyl acrylate), poly (isopropyl acrylate), poly (isobutyl acrylate), poly (octadecyl acrylate), poly (ethylene), poly (ethylene) low density, poly (ethylene) high density, poly (ethylene oxide), poly (ethylene terephthalate), poly (vinyl isobutyl ether), poly (vinyl acetate), poly (vinyl chloride), and polyurethane, and/or mixtures thereof.

Suitable pharmaceutically acceptable excipients include, but are not limited to, carriers, such as sodium citrate and dicalcium phosphate; fillers or extenders, such as stearates, silicas, gypsum, starches, lactose, sucrose, glucose, mannitol, talc, and silicic acid; binders, such as hydroxypropyl methylcellulose, hydroxymethyl-cellulose, alginates, gelatin, polyvinyl pyrrolidone, sucrose, and acacia; humectants, such as glycerol; disintegrating agents, such as agar, calcium carbonate, potato and tapioca starch, alginic acid, certain silicates, EXPLOTAB^{™}, crospovidone, and sodium carbonate; solution retarding agents, such as paraffin; absorption accelerators, such as quaternary ammonium compounds; wetting agents, such as cetyl alcohol and glycerol monostearate; absorbents, such as kaolin and bentonite clay; lubricants, such as talc, calcium stearate, magnesium stearate, solid polyethylene glycols, and sodium lauryl sulfate; stabilizers, such as fumaric acid; coloring agents; buffering agents; dispersing agents; preservatives; organic acids; and organic bases. The aforementioned excipients are given as examples only and are not meant to include all possible choices. Additionally, many excipients can have more than one role or function, or can be classified in more than one group; the classifications are descriptive only, and are not intended to limit any use of a particular excipient.

For example, a matrix-based dosage form can comprise N-2,3,3-tetramethylbicyclo-[2.1.1]heptan-2-amine; a filler, such as starch, lactose, or microcrystalline cellulose (AVICEL^{™}); a binder/controlled-release polymer, such as hydroxypropyl methylcellulose or polyvinyl pyrrolidone; a disintegrant, such as EXPLOTAB^{™}, crospovidone, or starch; a lubricant, such as magnesium stearate or stearic add; a surfactant, such as sodium lauryl sulfate or polysorbates; and a glidant, such as colloidal silicon dioxide (AEROSIL^{™}) or talc.

The amounts and types of polymers, and the ratio of water-soluble polymers to water-insoluble polymers in the inventive formulations are generally selected to achieve a desired release profile of N-2,3,3-tetramethylbicyclo-[2.1.1]heptan-2-amine, as described below, For example, by increasing the amount of water insoluble-polymer relative to the amount of water soluble-polymer, the release of the drug can be delayed or slowed. This is due, in part, to an increased impermeability of the polymeric matrix, and, in some cases, to a decreased rate of erosion during transit through the gastrointestinal tract.

Osmotic Pump Dosage Forms

In another embodiment, the modified release formulations of the present invention are provided as osmotic pump dosage forms. In an osmotic pump dosage form, a core containing N-2,3,3-tetramethylbicyclo-[2.1.1]heptan-2-amine and optionally one or more osmotic excipients is typically encased by a selectively permeable membrane having at least one orifice. The selectively permeable membrane is generally permeable to water, but impermeable to the drug. When the system is exposed to body fluids, water penetrates through the selectively permeable membrane into the core containing the drug and optional osmotic excipients. The osmotic pressure increases within the dosage form. Consequently, the drug is released through the orifice(s) in an attempt to equalize the osmotic pressure across the selectively permeable membrane.

In more complex pumps, the dosage form can contain two internal compartments in the core. The first compartment contains the drug and the second compartment can contain a polymer, which swells on contact with aqueous fluid. After ingestion, this polymer swells into the drug-containing compartment, diminishing the volume occupied by the drug, thereby delivering the drug from the device at a controlled rate over an extended period of time. Such dosage forms are often used when a zero order release profile is desired.

Osmotic pumps are well known in the art. For example, U.S. Pat. Nos. 4,088,864, 4,200,098, and 5.573.776, describe osmotic pumps and methods of their manufacture. The osmotic pumps useful in accordance with the present invention can be formed by compressing a tablet of an osmotically active drug, or an osmotically inactive drug in combination with an osmotically active agent, and then coating the tablet with a selectively permeable membrane which is permeable to an exterior aqueous-based fluid but impermeable to the drug and/or osmotic agent.

One or more delivery orifices can be drilled through the selectively permeable membrane wall. Alternatively, one or more orifices in the wall can be formed by incorporating leachable pore-forming materials in the wall. In operation, the exterior aqueous-based fluid is imbibed through the selectively permeable membrane wall and contacts the drug to form a solution or suspension of the drug. The drug solution or suspension is then pumped out through the orifice as fresh fluid is imbibed through the selectively permeable membrane.

Typical materials for the selectively permeable membrane include selectively permeable polymers known in the art to be useful in osmosis and reverse osmosis membranes, such as cellulose acylate, cellulose diacylate, cellulose triacylate, cellulose acetate, cellulose diacetate, cellulose triacetate, agar acetate, amylose triacetate, beta glucan acetate, acetaldehyde dimethyl acetate, cellulose acetate ethyl carbamate, polyamides, polyurethanes, sulfonated polystyrenes, cellulose acetate phthalate, cellulose acetate methyl carbamate, cellulose acetate succinate, cellulose acetate dimethyl aminoacetate, cellulose acetate ethyl carbamate, cellulose acetate chloracetate, cellulose dipalmitate, cellulose dioctanoate, cellulose dicaprylate, cellulose dipentanlate, cellulose acetate valerate, cellulose acetate succinate, cellulose propionate succinate, methyl cellulose, cellulose acetate p-toluene sulfonate, cellulose acetate butyrate, lightly cross-linked polystyrene derivatives, cross-linked poly(sodium styrene sulfonate), poly(vinylbenzyitrimethyl ammonium chloride), cellulose acetate, cellulose diacetate, cellulose triacetate, and/or mixtures thereof.

The osmotic agents that can be used in the pump are typically soluble in the fluid that enters the device following administration, resulting in an osmotic pressure gradient across the selectively permeable wall against the exterior fluid. Suitable osmotic agents include, but are not limited to, magnesium sulfate, calcium sulfate, magnesium chloride, sodium chloride, lithium chloride, potassium sulfate, sodium carbonate, sodium sulfite, lithium sulfate, potassium chloride, sodium sulfate, d-mannitol, urea, sorbitol, inositol, raffinose, sucrose, glucose, hydrophilic polymers such as cellulose polymers, and/or mixtures thereof.

As discussed above, the osmotic pump dosage form can contain a second compartment containing a swellable polymer. Suitable swellable polymers typically interact with water and/or aqueous biological fluids, which causes them to swell or expand to an equilibrium state. Acceptable polymers exhibit the ability to swell in water and/or aqueous biological fluids, retaining a significant portion of such imbibed fluids within their polymeric structure, so as to increase the hydrostatic pressure within the dosage form. The polymers can swell or expand to a very high degree, usually exhibiting a 2- to 50-fold volume increase. The polymers can be non-cross-linked or cross-linked. In one embodiment, the swellable polymers are hydrophilic polymers.

Suitable polymers include, but are not limited to, poly(hydroxy alkyl methacrylate) having a molecular weight of from 30,000 to 5,000,000 Daltons; kappa-carrageenan; polyvinylpyrrolidone having a molecular weight of from 10,000 to 360,000 Daltons; anionic and cationic hydrogels; polyelectrolyte complexes; poly(vinyl alcohol) having low amounts of acetate, cross-linked with glyoxal, formaldehyde, or glutaraldehyde, and having a degree of polymerization from 200 to 30,000 Daltons; a mixture including methyl cellulose, cross-linked agar and carboxymethyl cellulose; a water-insoluble, water-swellable copolymer produced by forming a dispersion of finely divided maleic anhydride with styrene, ethylene, propylene, butylene, or isobutylene; water-swellable polymers of N-vinyl lactams; and/or mixtures of any of the foregoing.

The term "orifice" as used herein comprises means and methods suitable for releasing the drug from the dosage form. The expression includes one or more apertures or orifices that have been bored through the selectively permeable membrane by mechanical procedures. Alternatively, an orifice can be formed by incorporating an erodible element, such as a gelatin plug, in the selectively permeable membrane. In such cases, the pores of the selectively permeable membrane form a "passageway" for the passage of the drug. Such "passageway" formulations are described, for example, in U.S. Pat. Nos. 3,845,770 and 3,916,899.

The osmotic pumps useful in accordance with this invention can be manufactured by known techniques. For example, the drug and other ingredients can be milled together and pressed into a solid having the desired dimensions (e.g., corresponding to the first compartment). The swellable polymer is then formed, placed in contact with the drug, and both are surrounded with the selectively permeable agent. If desired, the drug component and polymer component can be pressed together before applying the selectively permeable membrane: The selectively permeable membrane can be applied by any suitable method, for example, by molding, spraying, or dipping.

Membrane-Modified Dosage Forms

The modified release formulations of the present invention can also be provided as membrane-modified formulations. Membrane-modified formulations of the present invention can be made by preparing a rapid release core, which can be a monolithic (*e.g.*, tablet) or multi-unit (*e.g.*, pellet) type, and coating the core with a membrane. The membrane-modified core can then be further coated with a functional coating. In between the membrane-modified core and functional coating, a barrier or sealant can be applied. Details of membrane-modified, dosage forms are provided below.

For example, N-2,3,3-tetramethylbicyclo-[2.1.1]heptan-2-amine can be provided in a multiparticulate membrane-modified formulation. N-2,3,3-tetramethylbicyclo-[2.1.1]heptan-2-amine can be formed into an active core by applying the compound to a nonpareil seed having an average diameter in the range of about 0.4 to about 1.1 mm or about 0.85 to about 1.00 mm. The N-2,2,3-tetramethytbicyclo-[2.1.1]heptan-2-amine can be applied with or without additional excipients onto the inert cores, and can be sprayed from solution or suspension using a fluidized bed coater (*e.g.,* Wurster coating) or pan coating system. Alternatively, they can be applied as a powder onto the inert cores using a binder to bind the N-2,3,3-tetramethylbicyclo-[2.1.1]heptan-2-amine onto the cores. Active cores can also be formed by extrusion of the core with suitable plasticizers (described below) and any other processing aids as necessary.

The modified-release formulations of the present invention comprise at least one polymeric material, which is applied as a membrane coating to the drug-containing cores. Suitable water-soluble polymers include, but are not limited to, polyvinyl alcohol, polyvinylpyrrolidone, methylcellulose, hydroxypropylcellulose, hydroxypropylmethyl cellulose, polyethylene glycol, and/or mixtures thereof.

Suitable water-insoluble polymers include, but are not limited to, ethylcellulose, cellulose acetate, cellulose propionate, cellulose acetate propionate, cellulose acetate butyrate, cellulose acetate phthalate, cellulose triacetate, poly (methyl methacrylate), poly (ethyl methacrylate), poly (butyl methacrylate), poly (isobutyl methacrylate), and poly (hexyl methacrylate), poly (isodecyl methacrylate), poly (lauryl methacrylate), poly (phenyl methacrylate), poly (methyl acrylate), poly (isopropyl acrylate), poly (isobutyl acrylate), poly (octadecyl acrylate), poly (ethylene), poly (ethylene) low density, poly (ethylene) high density, poly (ethylene oxide), poly (ethylene terephthalate), poly (vinyl isobutyl ether), poly (vinyl acetate), poly (vinyl chloride), polyurethane, and/or mixtures thereof.

EUDRAGIT^{™} polymers (available from Rohm Pharma) are polymeric lacquer substances based on acrylates and/or methacrylates. A suitable polymer that is freely permeable to the active ingredient and water is EUDRAGIT^{™} RL. A suitable polymer that is slightly permeable to the active ingredient and water is EUDRAGIT^{™} RS. Other suitable polymers that are slightly permeable to the active ingredient and water, and exhibit a pH-dependent permeability include, but are not limited to, EUDRAGIT^{™} L, EUDRAGIT^{™} S, and EUDRAGIT^{™} E.

EUDRAGIT™ RL and RS are acrylic resins comprising copolymers of acrylic and methacrylic acid esters with a low content of quaternary ammonium groups. The ammonium groups are present as salts and give rise to the permeability of the lacquer films. EUDRAGIT^{™} RL and RS are freely permeable (RL) and slightly permeable (RS), respectively, independent of pH. The polymers swell in water and digestive juices, in a pH-independent manner. In the swollen state, they are permeable to water and to dissolved active compounds.

EUDRAGIT^{™} L is an anionic polymer synthesized from methacrylic acid and methacrylic acid methyl ester. It is insoluble in acids and pure water. It becomes soluble in neutral to weakly alkaline conditions. The permeability of EUDRAGIT^{™} L is pH dependent. Above pH 5.0, the polymer becomes increasingly permeable.

In one embodiment comprising a membrane-modified dosage form, the polymeric material comprises methacrylic acid co-polymers, ammonio methacrylate co-polymers, or a mixture thereof. Methacrylic acid co-polymers such as EUDRAGIT^{™} S and EUDRAGIT^{™} L (Rohm Pharma) are particularly suitable for use in the modified release formulations of the present invention. These polymers are gastroresistant and enterosoluble polymers. Their polymer films are insoluble in pure water and diluted acids. They dissolve at higher pHs, depending on their content of carboxylic acid. EUDRAGIT™ S and EUDRAGIT^{™} L can be used as single components in the polymer coating or in combination in any ratio. By using a combination of the polymers, the polymeric material can exhibit a solubility at a pH between the pHs at which EUDRAGIT^{™} L and EUDRAGIT^{™} S are separately soluble.

The membrane coating can comprise a polymeric material comprising a major proportion (*i.e.,* greater than 50% of the total polymeric content) of one or more pharmaceutically acceptable water-soluble polymers, and optionally a minor proportion (*i.e.,* less than 50% of the total polymeric content) of one or more pharmaceutically acceptable water-insoluble polymers. Alternatively, the membrane coating can comprise a polymeric material comprising a major proportion (*i.e*., greater than 50% of the total polymeric content) of one or more pharmaceutically acceptable water-insoluble polymers, and optionally a minor proportion (*i.e.,* less than 50% of the total polymeric content) of one or more pharmaceutically acceptable water-soluble polymers.

Ammonio methacrylate co-polymers such as EUDRAGIT^{™} RS and EUDRAGIT^{™} RL are suitable for use in the modified release formulations of the present invention. These polymers are insoluble in pure water, dilute acids, buffer solutions, or digestive fluids over the entire physiological pH range. The polymers swell in water and digestive fluids independently of pH. In the swollen state they are then permeable to water and dissolved actives. The permeability of the polymers depends on the ratio of ethylacrylate (EA), methyl methacrylate (MMA), and trimethylammonioethyl methacrylate chloride (TAMCI) groups in the polymer. Those polymers having EA:MMA:TAMCI ratios of 1:2:0.2 (EUDRAGIT^{™} RL) are more permeable than those with ratios of 1:2:0.1 (EUDRAGIT^{™} RS). Polymers of EUDRAGIT^{™} RL are insoluble polymers of high permeability. Polymers of EUDRAGIT^{™} RS are insoluble films of low permeability.

The ammonio methacrylate co-polymers can be combined in any desired ratio. For example, a ratio of EUDRAGIT^{™} RS:EUDRAGIT^{™} RL (90:10) can be used. The ratios can furthermore be adjusted to provide a delay in release of the drug. For example, the ratio of EUDRAGIT^{™} RS:EUDRAGIT^{™} RL can be about 100:0 to about 80:20, about 100:0 to about 90:10, or any ratio in between. In such formulations, the less permeable polymer EUDRAGIT^{™} RS would generally comprise the majority of the polymeric material.

The ammonio methacrylate co-polymers can be combined with the methacrylic acid co-polymers within the polymeric material in order to achieve the desired delay in release of the drug. Ratios of ammonio methacrylate co-polymer (*e.g*., EUDRAGIT^{™} RS) to methacrylic acid co-polymer in the range of about 99:1 to about 20:80 can be used. The two types of polymers can also be combined into the same polymeric material, or provided as separate coats that are applied to the core.

In addition to the EUDRAGIT^{™} polymers described above, a number of other such copolymers can be used to control drug release. These include methacrylate ester co-polymers (*e.g.*, EUDRAGIT^{™} NE 30D). Further information on the EUDRAGIT^{™} polymers can be found in "Chemistry and Application Properties of Polymethacrylate Coating Systems," in Aqueous Polymeric Coatings for Pharmaceutical Dosage Forms (ed. James McGinity, Marcel Dekker Inc., New York, pg 109-114).

The coating membrane can further comprise one or more soluble excipients so as to increase the permeability of the polymeric material. Suitably, the soluble excipient is selected from among a soluble polymer, a surfactant, an alkali metal salt, an organic acid, a sugar, and a sugar alcohol. Such soluble excipients include, but are not limited to, polyvinyl pyrrolidone, polyethylene glycol, sodium chloride, surfactants such as sodium lauryl sulfate and polysorbates, organic acids such as acetic acid, adipic acid, citric acid, fumaric acid, glutaric acid, malic acid, succinic acid, and tartaric acid, sugars such as dextrose, fructose, glucose, lactose and sucrose, sugar alcohols such as lactitol, maltitol, mannitol, sorbitol and xylitol, xanthan gum, dextrins, and maltodextrins. In some embodiments, polyvinyl pyrrolidone, mannitol, and/or polyethylene glycol can be used as soluble excipients. The soluble excipient(s) can be used in an amount of from about 1 % to about 10% by weight, based on the total dry weight of the polymer.

In another embodiment, the polymeric material comprises one or more water-insoluble polymers, which are also insoluble in gastrointestinal fluids, and one or more water-soluble pore-forming compounds. For example, the water-insoluble polymer can comprise a terpolymer of polyvinylchloride, polyvinylacetate, and/or polyvinylalcohol. Suitable water-soluble pore-forming compounds include, but are not limited to; saccharose, sodium chloride, potassium chloride, polyvinylpyrrolidone, and/or polyethyleneglycol. The pore-forming compounds can be uniformly or randomly distributed throughout the water-insoluble polymer. Typically, the pore-forming compounds comprise about 1 part to about 35 parts for each about 1 to about 10 parts of the water-insoluble polymers.

When such dosage forms come in to contact with the dissolution media (*e.g.*, intestinal fluids), the pore-forming compounds within the polymeric material dissolve to produce a porous structure through which the drug diffuses. Such formulations are described in more detail in U.S. Patent No. 4,557,925. The porous membrane can also be coated with an enteric coating, as described herein, to inhibit release in the stomach.

For example, a pore-forming modified-release dosage form can comprise N-2,3,3-tetramethylbicyclo-[2.1.1]heptan-2-amine; a filler, such as starch, lactose, or microcrystalline cellulose (AVICEL^{™}); a binder/modified release polymer, such as hydroxypropyl methylcellulose or polyvinyl pyrrolidone; a disintegrant, such as, EXPLOTAB^{™}, crospovidone, or starch; a lubricant, such as magnesium stearate or stearic acid; a surfactant, such as sodium lauryl sulphate or polysorbates; and a glidant, such as colloidal silicon dioxide (AEROSIL^{™)} or talc.

The polymeric material can also include one or more auxiliary agents such as fillers, plasticizers, and/or anti-foaming agents. Representative fillers include talc, fumed silica, glyceryl monostearate, magnesium stearate, calcium stearate, kaolin, colloidal silica, gypsum, micronized silica, and magnesium trisilicate. The quantity of filler used typically ranges from about 2% to about 300% by weight, and can range from about 20% to about 100%, based on the total dry weight of the polymer. In one embodiment, talc is the filler.

The coating membranes, and functional coatings as well, can also include a material that improves the processing of the polymers. Such materials are generally referred to as plasticizers and include, for example, adipates, azelates, benzoates, citrates; isoebucates, phthalates, sebacates, stearates, and glycols. Representative plasticizers include acetylated monoglycerides, butyl phthatyl butyl glycolate, dibutyl tartrate, diethyl phthalate, dimethyl phthalate, ethyl phthalyl ethyl glycolate, glycerin, ethylene glycol, propylene glycol, triacetin citrate, triacetin, tripropinoin, diacetin, dibutyl phthalate, acetyl monoglyceride, polyethylene glycols, castor oil, triethyl citrate, polyhydric alcohols, acetate esters, gylcerol triacetate, acetyl triethyl citrate, dibenzyl phthalate, dihexyl phthalate, butyl octyl phthalate, diisononyl phthalate, butyl octyl phthalate, dioctyl azelate, epoxidised tallate, triisoctyl trimellitate, diethylhexyl phthalate, di-n-octyl phthalate, di-i-octyl phthalate, di-i-decyl phthalate, di-n-undecyl phthalate, di-n-tridecyl phthalate, tri-2-ethylhexyl trimellitate, di-2-ethylhexyl adipate, di-2-ethylhexyl sebacate, di-2-ethylhexyl azelate, dibutyl sebacate, glyceryl monocaprylate, and glyceryl monocaprate. In one embodiment, the plasticizer is dibutyl sebacate. The amount of plasticizer used in the polymeric material typically ranges from about 10% to about 50%, for example, about 10, 20, 30, 40, or 50%, based on the weight of the dry polymer.

Anti-foaming agents can also be included. In one embodiment, the anti-foaming agent is simethicone. The amount of anti-foaming agent used typically comprises from about 0% to about 0.5% of the final formulation.

The amount of polymer to be used in the membrane-modified formulations is typically adjusted to achieve the desired drug delivery properties, including the amount of drug to be delivered, the rate and location of drug delivery, the time delay of drug release, and the size of the multiparticulates in the formulation. The amount of polymer applied typically provides an about 10% to about 100% weight gain to the cores. In one embodiment, the weight gain from the polymeric material ranges from about 25% to about 70%.

The combination of all solid components of the polymeric material, including co-polymers, fillers, plasticizers, and optional excipients and processing aids, typically provides an about 10% to about 450% weight gain on the cores. In one embodiment, the weight gain is about 30% to about 160%.

The polymeric material can be applied by any known method, for example, by spraying using a fluidized bed coater (*e.g.*, Wurster coating) or pan coating system. Coated cores are typically dried or cured after application of the polymeric material. Curing means that the multiparticulates are held at a controlled temperature for a time sufficient to provide stable release rates. Curing can be performed, for example, in an oven or in a fluid bed drier. Curing can be carried out at any temperature above room temperature.

A sealant or barrier can also be applied to the polymeric coating. A sealant or barrier layer can also be applied to the core prior to applying the polymeric material. A sealant or barrier layer is not intended to modify the release of N-2,3,3-tetramethylbicyclo-[2.1.1]heptan-2-amine. Suitable sealants or barriers are permeable or soluble agents such as hydroxypropyl methylcellulose, hydroxypropyl cellulose, hydroxypropyl ethylcellulose, and xanthan gum.

Other agents can be added to improve the processability of the sealant or barrier layer. Such agents include talc, colloidal silica, polyvinyl alcohol, titanium dioxide, micronized silica, fumed silica; glycerol monostearate, magnesium trisilicate, and magnesium stearate, or a mixture thereof. The sealant or barrier layer can be applied from solution (*e.g*., aqueous) or suspension using any known means, such as a fluidized bed coater (*e.g.*, Wurster coating) or pan coating system. Suitable sealants or barriers include, for example. OPADRY WHITE Y-1-7000 and OPADRY OY/B/28920 WHITE, each of which is available from Colorcon Limited, England.

The invention also provides an oral dosage form containing a multiparticulate N-2,3,3-tetramethylbicyclo-[2.1.1]heptan-2-amine formulation as hereinabove defined, in the form of caplets, capsules, particles for suspension prior to dosing, sachets, or tablets. When the dosage form is in the form of tablets, the tablets can be disintegrating tablets, fast dissolving tablets, effervescent tablets, fast melt tablets, and/or mini-tablets. The dosage form can be of any shape suitable for oral administration of a drug, such as spheroidal, cube-shaped, oval, or ellipsoidal. The dosage forms can be prepared from the multiparticulates in any known manner and can include additional pharmaceutically acceptable excipients.

All of the particular embodiments described above, including but not limited to, matrix-based, osmotic pump-based, soft gelatin capsules, and/or membrane-modified forms, which can further take the form of monolithic and/or multi-unit dosage forms, can have a functional coating. Such coatings generally serve the purpose of delaying the release of the drug for a predetermined period. For example, such coatings can allow the dosage form to pass through the stomach without being subjected to stomach acid or digestive juices. Thus, such coatings can dissolve or erode upon reaching a desired point in the gastrointestinal tract, such as the upper intestine.

Such functional coatings can exhibit pH-dependent or pH-independent solubility profiles. Those with pH-independent profiles generally erode or dissolve away after a predetermined period, and the period is generally directly proportional to the thickness of the coating. Those with pH-dependent profiles, on the other hand, can maintain their integrity while in the acid pH of the stomach, but quickly erode or dissolve upon entering the more basic upper intestine.

Thus, a matrix-based, osmotic pump-based, or membrane-modified formulation can be further coated with a functional coating that delays the release of the drug. For example, a membrane-modified formulation can be coated with an enteric coating that delays the exposure of the membrane-modified formulation until the upper intestine is reached. Upon leaving the acidic stomach and entering the more basic intestine, the enteric coating dissolves. The membrane-modified formulation then is exposed to gastrointestinal fluid, and releases N-2,3,3-tetramethylbicyclo-[2.1.1]heptan-2-amine over an extended period, in accordance with the invention. Examples of functional coatings such as these are known in the art.

The thickness of the polymer in the formulations, the amounts and types of polymers, and the ratio of water-soluble polymers to water-insoluble polymers in the modified-release formulations are generally selected to achieve a desired release profile of N-2,3,3-tetramethylbicyclo-[2.1.1]heptan-2-amine. For example, by increasing the amount of water-insoluble-polymer relative to the water-soluble polymer, the release of the drug can be delayed or slowed.

Any formulation of the present invention can also contain a suitable compound that enhances the absorption of N-2,3,3-tetramethylbicyclo-[2.1.1]heptan-2-amine. These enhancers include, but are not limited to, cell envelope disordering compounds, solvents, steroidal detergents, bile salts, chelators, surfactants, non-surfactants, fatty acids, and mixtures thereof. The organic solvent can be selected from, but is not limited to, a C₂ or C₃ alcohol, a C₃ or C₄ diol, dimethylsulfoxide, N,N-dimethylformamide, 1-n-dodecylcyclazacyclo-heptan-2-one, N-methyl pyrrolidone, N-(2-hydroxyethyl) pyrrolidone, triacetin, propylene carbonate and dimethyl isosorbide and mixtures thereof. The cell-envelope disordering compounds that can be used include, but are not limited to, isopropyl myristate, methyl laurate, oleic acid, oleyl alcohol, glycerol monooleate, glycerol dioleate, glycerol trioleate, glycerol monostearate, glycerol monolaurate, propylene glycol monolaurate, sodium dodecyl sulfate, and sorbitan esters and mixtures thereof. Bile salts that can be used include, but are not limited to, natural and synthetic salts of cholanic acid and mixtures thereof.

The amount of the dose administered, as well as the dose frequency, will vary depending on the particular dosage form used and route of administration. The amount and frequency of administration will also vary according to the age, body weight, and response of the individual subject. Typical dosing regimens can readily be determined by a competent physician without undue experimentation. It is also noted that the clinician or treating physician will know how and when to interrupt, adjust, or terminate therapy in conjunction with individual subject response.

In general, the total daily dosage for reducing, preventing, and/or managing the abnormal increases in gastrointestinal motility and/or the intestinal conditions that cause the same, with any of the formulations according to the present invention, is from about 0.2 mg to about 40 mg, or from about 0.5 mg to about 20 mg, or from about 1 mg to about 15 mg, or from about 2 mg to about 12 mg, or any amount in between, of N-2,3,3tetramethylbicyclo-[2.1.1]heptan-2-amine, or a pharmaceutically acceptable salt thereof. For example, for an orally administered dosage form, the total daily dose can range from about 0.5 mg to about 20 mg, or from about 1 mg to about 15 mg, or from 2 mg to about 12 mg. Accordingly, a single oral dose can be formulated to contain about 0.2 mg, 0.5 mg, 1 mg, 2 mg, 2.5 mg, 3 mg, 4 mg, 5 mg, 6 mg, 8 mg , 10 mg, 12 mg, 15 mg, 20 mg, or any amount in between, of N-2,3,3-tetramethylbicyclo-[2.1.1]heptan-2-amine, or a pharmaceutically acceptable salt thereof.

In the case of transdermal formulations, an excess of N-2,3,3-tetramethylbicyclo-[2.1.1]heptan-2-amine can be incorporated into the transdermal system in order to ensure an effective concentration gradient for transdermal absorption. Thus transdermal units can contain from about 0.2 mg to about 120 mg, or from about 0.5 mg to about 100 mg, or from about 1 mg to about 80 mg, or from about 2 mg to about 60 mg, or any amount in between, of N-2,3,3-tetramethylbicyclo-[2.1.1]heptan-2-amine.

The pharmaceutical formulations described herein can be formulated such that the maximum plasma concentration of N-2,3,3-tetromethylbicyclo-[2.1.1]heptan-2-amine can be achieved at about 3.5, 4, 5, 6, 7, 8, 9, 10, 12, 14, 18, or 24 hours, or anytime in between, following a first administration of the formulation of the present invention.

The pharmaceutical formulations containing N-2,3,3-tetramethylbicyclo-[2.1.1]heptan-2-amine, or a pharmaceutically acceptable salt thereof, can be administered in single or in divided doses, 1, 2, 3, 4, 5, or more times each day. Alternatively, the dose can be delivered one or more times every 2, 3, 4, 5, 6, 7, or more days. In one embodiment, the pharmaceutical formulations are administered once per day.

Any of the pharmaceutical formulations and dosage forms described herein can further comprise one or more pharmaceutically active compounds other than N-2,3,3-tetramethylbicyclo-[2.1.1]heptan-2-amine, or a pharmaceutically acceptable salt thereof. Such compounds can be included to treat, prevent, and/or manage an increase in gastrointestinal motility being reduced, prevented, and/or managed with N-2,3,3-tetramethylbicyclo-[2.1.1]heptan-2-amine, or a pharmaceutically acceptable salt thereof, or a different one. Those of skill in the art are familiar with examples of the techniques for incorporating additional active ingredients into formulations comprising N-2,3,3-tetramethylbicyclo-[2.1.1]heptan-2-amine, or a pharmaceutically acceptable salt thereof. Alternatively, such additional pharmaceutically active compounds can be provided in a separate formulation and co-administered to a subject with a formulation according to the present invention. Such separate formulations can be administered before, after, or simultaneously with the administration of formulations of the present invention containing N-2,3,3-tetramethylbicyclo-[2.1.1]heptan-2-amine, or a pharmaceutically acceptable salt thereof. The additional pharmaceutically active compounds that can be used include, but are not limited to, other ganglionic blockers and/or nicotinic-receptor antagonists (such as hexamethonium, trimethaphan, chloroisondamine, erysodine, β-dihydroerythrodine, amantidine, perpidine, succinylcholine, decamethonium, tubocurarine (including isomers thereof such as d-tubocurarine), atracurium, doxacurium, mivicurium, pancuronium, rocuronium, and vencuronium, for example), agents that alter gastrointestinal motility, antispasmodics. antimuscarinic agents, glycopyrrolate, atropine, hyscomine, scopolamine, opiates (such as loperamide, difenoxine, codeine, morphine, oxymorphone, oxycontin, dihydrocodeine, and fentanyl, for example), 5-HT receptor agonists, 5-HT antagonists (such as alosetron hydrochloride, for example), calcium channel blockers (such as verapamil, including its intestinal selective isomers, for example), beta blockers (including beta blockers having effects on gastrointestinal function through neurogenic activity), agents used to treat various gastrointestinal symptoms and diseases including those that alter fluid transport across the gut or into or out of gastrointestinal cells, diuretics (such as amiloride and furosemide, for example), anti-diarrheals (such as bismuth and sandostatin, for example), H₂-antihistamines, proton pump inhibitors, antacids, anti-inflammatory agents, sulfasalazine, steroids (such as mineralocorticoids, corticosteroids, estrogens, prednisone, prednisolone, cortisol, cortisone, fluticasone, dexamethasone, and betamethasone, for example), 5-aminosalicylic acid, anti-infective agents (such as metronidazole, ciprofloxacin, and azathioprine, for example), immunomodulators (such as 6-mercaptopurine, cyclosporine, and methotrexate, for example), fish oil, remicade, heparin, and nicotine.

The invention is further illustrated by reference to the following examples.

**EXAMPLES**

Example 1: Activity of N-2,3,3-tetramethylbicyclo-[2.1.1]heptan-2-amine in the colon

Subjects diagnosed with increased gastrointestinal motility, due to irritable bowel syndrome for example, are recruited into a clinical study examining the effects of N-2,3,3-tetramethylbicyclo-[2.1.1]heptan-2-amine, or a pharmaceutically acceptable salt thereof. Gastrointestinal transit times are measured by a standard technique, for example the hydrogen breath test following lactulose administration (Miller et al., Dig. Dis. Sd., 42:10-18, 1997). Measurements are made before and after administration of an effective dose of N-2,3,3-tetramethylbicyclo-[2.1.1]heptan-2-amine, or a pharmaceutically acceptable salt thereof. Typical pre-treatment values of transit times are of the order of 70 minutes. Following administration of N-2,3,3-tetramethylbicyclo-[2.1.1]heptan-2-amine, or a pharmaceutically acceptable salt thereof, the values of transit time increase toward the normal range, on the order of 90 minutes.

In a further study, subjects diagnosed with diarrhea-predominant irritable bowel syndrome are recruited Into a study to measure rectal sensitivity. A balloon is located in the rectum and the volume is gradually increased until the subject feels the need to defecate. This measurement is made before and after the administration of an effective dose of N-2,3,3-tetramethylbicyclo-[2.1.1]heptan-2-amine, or a pharmaceutically acceptable salt thereof. Subjects with diarrhea-predominant IBS typically have a lower threshold to discomfort upon intrarectal balloon distension than normal control subjects. (Naliboff B.D. et al., Gut, 41: 505-512, 1997). The administration of N-2,3,3-tetramethylbicyclo-[2.1.1]heptan-2-amine amine, or a pharmaceutical acceptable salt thereof, increases this threshold toward normal values.

Example 2: Production of Modified-Release Tablet Formulations of N-2,3,3-tetramethybicyclo-[2.1.1]heptan-2-amine with Varying Amounts and Grades of Methocel^{™} using Wet Granulation

| Ingredient | FUNCTION | Qty % (w/w) | Qty % (w/w) | Qty % (w/w) |
|---|---|---|---|---|
| N-2,3,3-tetramethylbicyclo-[2.1.1] heptan-2-amine | Active | 6.0 | 6.0 | 6.0 |
| LACTOSE | Diluent | 47.85 | 33.81 | 19.31 |
| AVICEL^{™} PH101 | Dry Binder diluent | 20.45 | 14.49 | 8.99 |
| METHOCEL^{™} | Modified Release Polymer | 20.0 | 40.0 | 60.0 |
| COLLOIOAL SILICON DIOXIDE | Glidant | 0.2 | 0.2 | 0.2 |
| MAGNESIUM STEARATE | Lubricant | 0.5 | 0.5 | 0.5 |
| POLYVINYL PYRROLIDONE (PVP) | Binder | 5.0 | 5.0 | 5.0 |
| *ISOPROPYL ALCOHOL (IPA) | Solvent | N/A | N/A | N/A |
| TOTAL | | 100 | 100 | 100 |

| | | | | |
|---|---|---|---|---|
| *Removed during processing. | | | | |

Each of the above-listed ingredients is weighed. The PVP is dissolved in the IPA to form a PVP solution. The N-2,3,3-tetramethylbicyclo-[2.1.1]heptan-2-amine is mixed with the Methocel^{™}, 50% of the Avicel^{™}, and 50% of the lactose in a suitable mixer (*e.g*., Planetary (Hobart), High Shear (Diosna/Fielder)) for 15 min to produce a homogenous mixture. The Methocel^{™} can be substituted with various grades, such as the K and/or E Series, as described by the manufacturer (Dow Chemicals). White continuing to mix, the granulating fluid (PVP Solution) is added. This combination is mixed until a desired granulation end point is achieved (add more IPA if needed to produce a suitable granule). The granules are dried with suitable drying equipment (*e.g.*, oven or fluidization equipment) until an acceptable level of moisture (*e.g.*, <1.0%) and IPA (*e.g.*, <0.5%) is achieved.

The dry granulate is then passed through suitable comminution equipment (*e.g*., Co-Mill, Fitzpatrick mill) fitted with a suitable sized screen (100-500 micron). The granulate is mixed with the colloidal silicon dioxide, sodium starch glycolate, and the remainder of the lactose and Avicel^{™} in a blender for 15 min. The magnesium stearate is added, and the mixture is mixed for an additional 5 min. The resulting mixture is compressed into oval shaped tablets to a target weight.

Example 3: Production of Immediate-Release Drug Loaded Multiparticulate Formulations of N-2,3,3-tetramethylbicyclo-[2.1.1]heptan-2-amine

| Ingredient | FUNCTION | Qty (mg/g) |
|---|---|---|
| N-2,3,3-tetramethylbicyclo-[2.1.1]heptan-2-amine | Active | 30.0 |
| NON PAREIL SEEDS | Inert carrier | 770.0 |
| POLYVINYL PYRROLIDONE (PVP) | Binder | 50.0 |
| TALC | Anti-adherent | 125.0 |
| COLLOIDAL SILICON DIOXIDE | Glidant | 25.0 |
| WATER | Solvent | N/A |
| TOTAL | | 1000 |

The N-2,3,3-tetramethylbicyclo-[2.1.1]heptan-2-amine, binder, glidant, and anti-adherent are dissolved and/or suspended in water. The suspension is then sprayed onto the nonpareil seeds using an appropriate fluidized coating machine (*e.g.*, Glatt apparatus). After the solution suspension has been applied to the nonpareil seeds, the drug-loaded immediate-release multiparticulates are dried in the fluidized coating machine.

The drug-loaded immediate-release multiparticulates can then be further processed into a modified-release formulation, as described below. In addition, the drug loaded immediate release multiparticulates can be used in combination with the modified-release multiparticulates described in Example 8, depending on the release profile that is desired.

Example 4: Production of a Modified-Release Multiparticulate Formulation of N-2,3,3-tetramethylbicyclo-[2.1.1]heptan-2-amine

Immediate-release drug-loaded multiparticulates of N-2,3,3-tetramethylbicyclo-[2.1.1]heptan-2-amine are prepared, as described above. The multiparticulates are then coated with polymer solution A or B, as follows, to produce a modified-release multiparticulate formulation.

**Polymer Solution A**

| Ingredient | FUNCTION | Batch (g) |
|---|---|---|
| EUDRAGIT^{™} RS 30D | Modified Release Polymer | 200.0 |
| TALC | Anti-adherent | 60.0 |
| TRIETHYL CITRATE | Plasticizer | 12.0 |
| SIMETHICONE EMULSION | Dispersant | 1.0 |
| WATER | Solvent | 392.0 |
| TOTAL | | 665.00 |

**Polymer Solution B**

| Ingredient | FUNCTION | Batch (g) |
|---|---|---|
| EUDRAGIT^{™} RS 12.5 | Modified Release Polymer | 900.0 |
| EUDRAGIT^{™} RL 12.5 | Modified Release Polymer | 300.0 |
| TALC | Anti-adherent | 105.0 |
| DIBUTYL SEBECATE | Plasticizer | 15.0 |
| MAGNESIUM STEARATE | Anti-adherant | 30.0 |
| ACETONE | Solvent | 825.0 |
| ISOPROPYL ALCOHOL (IPA) | Solvent | 825.0 |
| TOTAL | | 3000.00 |

The above listed ingredients in each table are mixed to produce polymer solutions A and B, respectively.

| Ingredient | FUNCTION | Batch (g) | Batch (g) | Batch (g) |
|---|---|---|---|---|
| N-2,3,3- tetramethylbicyclo-[2.1.1]heptan-2-amine | Active agent with carrier and excipients | 1000 | 1000 | 1000 |
| *Polymer Solution A or B | Modified Release Polymer | 50 | 100 | 200 |
| TOTAL | | 1050 | 1100 | 1200 |

| | | | | |
|---|---|---|---|---|
| *Represents the amount of solid content in polymer solution A or B as the water is removed during processing. The amount of solids applied can be adjusted depending on the type of dissolution profile that is required. Increased amounts of polymer solids will produce decreasing dissolution profiles. | | | | |

The compound-loaded immediate-release multiparticulates are placed in a suitable fluidized coating machine (*e.g.*, Glatt apparatus). The polymer solution (polymer solution A or B) is then sprayed onto the compound-loaded immediate-release multiparticulates in the amounts indicated above. After the required amount of polymer solution has been applied, the polymer-coated multiparticulates are dried in the fluidized coating machine. The resulting modified-release multiparticulates are encapsulated into a hard gelatin capsule using an automated encapsulation machine, in an amount sufficient to produce a 0.2, 0.5, 1.0, 2.0, 2.5, 3, 4, 5, 6, 8, 10, 12, 15, 18, 20, or 24 mg dose.

Example 5: Production of Adhesive Diffusion Controlled Transdermal Formulations

| Ingredient | FUNCTION | Qty % (w/w) | Qty % (w/w) | Qty % (w/w) |
|---|---|---|---|---|
| N-2,3,3-tetramethylbicyclo-[2.1.1]heptan-2-amine | Active | 2 | 3 | 2 |
| Acrylic Adhesive | Adhesive Polymer Matrix | 53 | 52 | |
| Silicone Adhesive | Adhesive Polymer Matrix | | | 53 |
| Heptane | Solvent | | | Residual levels only |
| Cyclohexhane | Solvent | Residual levels only | | |
| Hexhane | Solvent | | Residual levels only | |
| Polyester Backing | Impermeable Backing Layer | 12% | 12% | 12% |
| Polyester Release Liner | Disposable Release Liner | 33% | 33% | 33% |
| TOTAL | | 100 | 100 | 100 |

The N-2,3,3-tetramethylbicyclo-[2.1.1]heptan-2-amine is mixed with the dissolved adhesive polymer in a solvent. This mixture is coated onto the polyester backing layer at 10mg/cm² and dried at about 30-50°C for about 5 min. This layer, when dried to below the specified residual solvent levels, is then laminated to a release liner to form a 3-layer system. Additional adhesive layers can be added to modify the release profile. Individual transdermal patches are cut from this system with the strength and administered dose reflecting the surface area of the cut patch. The in vitro flux across skin, i.e., the permeation rate, is measured with a sample of human cadaver skin in a modified Franz diffusion cell. Human epidermal cells having a receiving volume of 7 mL and an active area of 0.64 cm² are used. The receiving compartment of the cells is filled with ammonium phosphate buffer at pH 4.0 to pH 7.0, stirred at 300 RPM, and the temperature is maintained at 32°C. Human cadaver skin is obtained from a skin bank in "pre-peeled" condition with the stratum corneum removed. The skin is stored in an ammonium phosphate solution until use. Skin punches are placed on the diffusion cells with the outer skin layers facing the donor compartment. A transdermal patch of the present invention is applied to the skin and pressed to cause uniform contact with the skin and the skin is placed across the orifice of the receiving compartment of the diffusion cell. The cell is placed in a chamber with a constant temperature of about 32°C and a relative humidity of about 45%. The receiving fluid is stirred by means of a magnetic stirrer throughout the experiment to assure a uniform sample and a reduced diffusion barrier on the dermal side of the skin. The entire volume of receptor fluid is withdrawn at about 3, 6, 12, 24, 36 and 48 hours and immediately replaced with fresh fluid. The withdrawn fluid is analyzed for N-2,3,3-tetramethylbicyclo-[2.1.1]heptan-2-amine content using conventional high performance liquid chromatography or other appropriate analytical methods. The permeation rate is calculated from the slope of the cumulative amounts of N-2,3,3-tetramethylbicyclo-[2.1.1]heptan-2-amine in the receiver compartment versus time.

Example 6: Biostudy

An open label, single dose, 4-treatment, four period, balanced, randomized, crossover study is designed to compare and assess the relative bioavailability of three modified-release formulations with an immediate-release form (Inversine 2.5 mg tablets, Targacept). Modified-release formulations are prepared as follows: 1) a modified-release tablet according to Example 2; 2) a modified-release multiparticulate capsule form according to Example 4; and 3) a modified-release transdermal form according to Example 5.

Sixteen healthy volunteers are dosed on each of four occasions with at least a seven-day washout period between each dose. The volunteers are fasting from food and beverages other than water for at least 4 hours before dosing in each treatment period. Water is proscribed for one hour before and one hour after dosing except for the 150 mL of water at the time of dosing. Venous blood samples are obtained at regular time intervals immediately prior to and following each dosing for a period of 48 hours. Concentrations of N-2,3,3-tetramethylbicyclo-[2.1.1]heptan-2-amine in plasma are measured. Individual plasma concentration curves are constructed and individual, mean, and relative pharmacokinetic parameters are estimated including Tmax, Cmax, AUC, and FI.

Example 7: Treatment of Irritable Bowel Syndrome (lBS) with N-2,3,3-tetramethylbicyclo-[2.1.1]hentan-2-amine

Modified-release formulations according to Examples 2, 4, and 5 are prepared. A subject is diagnosed with diarrhea-dominant IBS by meeting the ROME II criteria for IBS. the criteria requires at least 12 weeks, which need not be consecutive, in the preceding 12 months, of abdominal discomfort or pain that has at least two out of three features: 1) relief with defectation; and/or 2) onset associated with a change in frequency of stool; and/or 3) onset associated with a change in appearance of the stool. Additional, the criteria requires one or more of the following symptoms: 1) more than three bowel movements a day, 2) loose or watery stools; and 3) an urgency to defecate combined with no fewer than three bowel movements a week, no hard or lumpy stools, and no straining during a bowel movement.

Such a subject receives a daily administration of N-2,3,3-tetramethylbicyclo-[2.1.1]heptan-2-amine, or a pharmaceutically acceptable salt thereof, in a modified-release form, at an initial dose of about 2.5 mg for about 4 weeks. The subject's global assessment of symptoms and abdominal discomfort/pain is used to measure the improvement in symptoms. Safety is assessed including monitoring for effects on blood pressure and heart rate. If side effects are limiting the dose can be reduced. Once the effect and acceptable safety of the 2.5 mg dose is established, the dose can be safely titrated to higher levels at intervals of about 4 weeks.

The formulations of this example demonstrate efficacy in improving IBS symptoms. Additionally, they demonstrate a dissociation of gastrointestinal motility effects from effects on other systems, including blood pressure, heart rate, vision, and bladder function.

## Claims

1. Use of N-2,3,3-tetramethylbicyclo-[2.1.1]heptan-2-amine, or a pharmaceutically acceptable salt thereof for the manufacture of a medicament for reducing gastrointestinal motility in a subject suffering from an abnormal increase in gastrointestinal motility wherein said N-2,3,3-tetramethylbicyclo-[2.1.1]heptan-2-amine comprises racemic N-2,3,3-tetramethylbicyclo-[2.1.1]heptan-2-amine, enriched (R)-N-2,3,3-tetramethylbicyclo-[2.1.1]heptan-2-amine, or enriched (S)-N-2,3,3-tetramethylbicyclo-[2.1.1]heptan-2-amine, and wherein said abnormal increase in gastrointestinal motility is caused by at least one condition chosen from inflammatory bowel disease, ulcerative colitis, granulomatous enteritis, infectious diseases of the small or large intestine, pyloric spasm, abdominal cramps, a functional bowel disorder, mild dysenteries, diverticulitis, acute enterocolitis, neurogenic bowel disorders, splenic flexure syndrome, neurogenic colon, spastic colitis, or is a symptom of any of the foregoing conditions.

2. Use according claim 1, wherein the condition is a functional bowel disorder, or irritable bowel syndrome.

3. Use according to claim 1, wherein in use said abnormal increase in gastrointestinal motility is reduced while minimizing at least one side effect associated with the administration of a conventional formulation of N-2,3,3-tetramethylbicyclo-[2.1.1]heptan-2-amine, or a pharmaceutically acceptable salt thereof.

4. Use according to claim 3, wherein said at least one effect is chosen from effects on said subject's heart rate, blood pressure, vision, and bladder function.

5. Use according to claim 1, wherein in use said N-2,3,3-tetramethylbicyclo-[2.1.1]heptan-2-amine, or a pharmaceutically acceptable salt thereof, is administered in the form of a pharmaceutically acceptable formulation.

6. Use according to claim 5, wherein said pharmaceutically acceptable formulation is a modified-release formulation.

7. Use according to claim 5, wherein said pharmaceutically acceptable formulation comprises a modified-release formulation in combination with an immediate-release formulation.

8. Use according to any of claims 5-7, wherein said pharmaceutically acceptable formulation is suitable for oral, intra-nasal, or transdermal administration.

9. Use according to claim 8, wherein said pharmaceutically acceptable formulation is suitable for buccal or sublingual administration.

10. Use according to claim 1, wherein said N-2,3,3-tetramethylbicyclo-[2.1.1]heptan-2-amine or a pharmaceutically acceptable salt thereof is present in an amount of about 0.2 mg to about 40 mg, preferably in an amount of about 0.5 mg to about 20 mg, more preferably in an amount of about 1 mg to about 15 mg, particularly preferably in an amount of about 2 mg to about 12 mg.

11. Use according to claim 1, wherein in use administration of N-2,3,3-tetramethylbicyclo-[2.1.1]heptan-2-amine, or a pharmaceutically acceptable salt thereof, provides a maximum plasma concentration of N-2,3,3-tetramethylbicyclo-[2.1.1]heptan-2-amine at about 3.5 hours or later following a first administration, preferably at about 6 hours or later following a first administration.

12. Use according to claim 1, wherein in use said N-2,3,3-tetramethylbicyclo-[2.1.1]heptan-2-amine, or a pharmaceutically acceptable salt thereof, is administered once-daily.

13. Use according to claim 1, wherein said N-2,3,3-tetramethylbicyclo-[2.1.1]heptan-2-amine comprises racemic N-2.3,3-tetramethylbicyclo-[2.1.1]heptan-2-amine, or a pharmaceutically acceptable salt thereof.

14. Use according to claim 1, wherein said N-2,3.3-tetramethylbicyclo-[2.1.1]heptan-2-amine comprises enriched N-2,3,3-tetramethylbicyclo-[2.1.1]heptan-2-amine, or a pharmaceutically acceptable salt thereof.

15. Use according to claim 1, wherein in use said N-2,3,3-tetramethylbicyclo-[2.1.1]heptan-2-amine, or a pharmaceutically acceptable salt thereof, is administered in combination with at least one other pharmaceutical active compound.

16. Use according to claim 15, wherein said at least one other pharmaceutically active compound is chosen from ganglionic blockers, nicotinic-receptor antagonists, gastrointestinal motility altering agents, antispasmodics, antimuscarinic agents, opiates, 5-HT receptor agonists, 5-HT receptor antagonists, calcium channel blockers, beta adrenergic receptor blockers, agents that alter fluid transport across the gut, agents that alter fluid transport into or out of gastrointestinal cells, diuretics, anti-diarrheals, H₂-antihistamines, proton pump inhibitors, antacids, anti-inflammatory agents, steroids, mineralocorticoids, corticosteroids, anti-infective agents, immunomodulators, and fish oil.

17. Use according to claim 16, wherein said at least one other pharmaceutically active compound is chosen from hexamethonium, trimethaphan, chloroisondamine, erysodine, β-dihydroerythrodine, amantidine, perpidine, succinylcholine, decamethonium, tubocurarine, atracurium, doxacurium, mivicurium, pancuronium, rocuronium, vencuronium, glycopyrrolate, atropine, hyscomine, scopolamine, loperamide, difenoxine, codeine, morphine, oxymorphone, oxycontin, dihydrocodeine, fentanyl, alosetron hydrochloride, verapamil, amiloride, furosemide, bismuth, sandostatin, sulfasalazine, estrogens, prednisone, prednisolone, cortisol, cortisone, fluticasone, dexamethasone, betamefhasone, 5-aminosalicylic acid, metronidazole, ciprofloxacin, azathioprine, 6-mercaptopurine, cyclosporine, methotrexate, fish oil, remicade, heparin, and nicotine.

18. Use according to claim 8, wherein said formulation comprises extended-release components, or delayed-release components, or both extended-release and delayed-release components.

19. Use according to claim 8, wherein in use said formulation produces a peak:trough plasma level ratio of N-2,3,3-tetramethylbicyclo-[2.1.1]heptan-2-amine of less than about 4:1, preferably less than about 3:1, more preferably less than about 2:1.

20. Use according to claim 8, wherein administration of said formulation provides a plasma concentration of N-2,3,3-tetramethylbicyclo[2.1.1]heptan-2-amine, at least about 24 hours following a first administration, that is greater than or equal to about 25% of the peak plasma concentration achieved following said administration, preferably wherein said plasma concentration of N-2,3,3-tetramethylbicyclo[2.2.1]heptan-2-amine, at least about 24 hours following a first administration, is greater than or equal to about 50% of the peak plasma concentration achieved following said administration.

21. Use according to claim 8; wherein administration of said formulation provides a plasma concentration of N-2,3,3-tetramethylbicyclo-[2.1.1]heptan-2-amine that is greater than or equal to about 50% of the peak plasma concentration, for greater than or equal to about 14 hours, following a first administration, preferably greater than or equal to about 50% of the peak plasma concentration, for greater than or equal to about 16 hours, following a first administration.

22. Use according to claim 21, wherein said plasma concentration of N-2,3,3-tetramethylbicyclo-[2.1.1]heptan-2-amine is greater than or equal to about 50% of the peak plasma concentration, for greater than or equal to about 18 hours, following a first administration, preferably greater than or equal to about 50% of the peak plasma concentration, for greater than or equal to about 24 hours, following a first administration.

23. Use according to claim 8, wherein the formulation, when tested in a U.S. Pharmacopeia (USP) Type 2 Apparatus, at 37°C, stirred at 50 rpm, and in pH 6.8 phosphate buffer, releases less than about 50% of said N-2,3,3-tetramethylbicyclo[2.1.1]heptan-2-amine in less than about 2 hours, greater than or equal to about 40% in about 12 or more hours, and about 70% or more in about 24 or more hours.

24. Use according to claim 23, wherein less than or equal to about 50% of said N-2,3,3-tetramethylbicyclo[2.1.1]heptan-2-amine is released in about 2 hours, less than or equal to about 70% is released in about 4 hours, greater than or equal to about 50% is released in about 8 hours, greater than or equal to about 65% is released in about 12 hours, and greater than or equal to about 80% is released in about 24 hours.

25. Use according to claim 24, wherein less than or equal to about 40% of said N-2,3,3-tetramethylbicyclo [2.1.1]heptan-2-amine is released in about 2 hours, less than or equal to about 65% is released in about 4 hours, greater than or equal to about 60% is released in about 8 hours, greater than or equal to about 70% is released in about 12 hours, and greater than or equal to about 80% is released in about 24 hours.

26. Use according to claim 25, wherein less than or equal to about 30% of said N-2,3,3-tetramethylbicyclo[2.1.1]heptan-2-amine is released in about 2 hours, about 20% to about 60% is released in about 4 hours, greater than or equal to about 70% is released in about 8 hours, greater than or equal to about 75% is released in about 12 hours, and greater than or equal to about 80% is released in about 24 hours.

27. Use according to claim 8, wherein said formulation comprises racemic N-2,3,3-tetramethylbicyclo-[2.1.1]heptan-2-amine, or a pharmaceutically acceptable salt thereof.

28. Use according to claim 8, wherein said formulation comprises enriched (R)-N-2,3,3-tetramethylbicyclo-[2.1.1]heptan-2-amine, or a pharmaceutically acceptable salt thereof.

29. Use according to claim 8, wherein said formulation comprises enriched (S)-N-2,3,3-tetramethylbicyclo-[2.1.1]heptan-2-amine, or a pharmaceutically acceptable salt thereof.

30. Use according to claim 8, wherein said formulation comprises at least one other pharmaceutically active compound.

31. Use according to claim 30, wherein said at least one other pharmaceutically active compound is chosen from ganglionic blockers, nicotinic-receptor antagonists, gastrointestinal motility altering agents, antispasmodics, antimuscarinic agents, opiates, 5-HT receptor agonists, 5-HT receptor antagonists, calcium channel blockers, beta adrenergic receptor blockers, agents that alter fluid transport across the gut, agents that alter fluid transport into or out of gastrointestinal cells, diuretics, anti-diarrheals, H₂-antihistamines, proton pump inhibitors, antacids, anti-inflammatory agents, steroids, mineralocorticoids, corticosteroids, anti-infective agents, immunomodulators, and fish oil.

32. Use according to claim 31, wherein said at least one other pharmaceutically, active compound is chosen from hexamethonium, trimethaphan, chloroisondamine, erysodine, β-dihydroerythrodine, amantidine, perpidine, succinylcholine, decamethonium, tubocurarine, atracurium, doxacurium, mivicurium, pancuronium, rocuronium, vencuronium, glycopyrrolate, atropine, hyscomine, scopolamine, loperamide, difenoxine, codeine, morphine, oxymorphone, oxycontin, dihydrocodeine, fentanyl, alosetron hydrochloride, verapamil, amiloride, furosemide, bismuth, sandostatin, sulfasalazine, estrogens, prednisone, prednisolone, cortisol, cortisone, fluticasone, dexamethasone, betamethasone, 5-aminosalicylic acid, metronidazole, ciprofloxacin, azathioprine. 6-mercaptopurine, cyclosporine, methotrexate, fish oil, remicade, heparin, and nicotine.

33. Use according to claim 5, wherein the formulation is a transdermal formulation that, when tested using modified Franz diffusion cells of human epidermis, in phosphate buffer at a pH of about 4 to about 7, at about 32°C, exhibits a permeation rate in which less than about 50% of the N-2,3,3-tetramethylbicyclo[2.1.1]heptan-2-amine is released in less than about 2 hours, greater than or equal to about 40% is released in about 12 or more hours, and about 70% or more is released in about 24 or more hours.

34. Use according to claim 33 wherein less than or equal to about 40% of said N-2,3,3-tetramethylbicyclo [2.1.1] heptan-2-amine is released in about 2 hours, about 10% to about 70% is released in about 4 hours, about 20% to about 80% is released in about 8 hours, greater than or equal to about 40% is released in about 12 hours, and greater than or equal to about 70% is released in about 24 hours.

35. Use according to claim 34, wherein less than or equal to about 30% of said N-2,3,3-tetramethylbicyclo[2.1.1]heptan-2-amine is released in about 2 hours, about 15% to about 60% is released in about 4 hours, about 30% to about 70% is released in about 8 hours, greater than or equal to about 50% is released in about 12 hours, and greater than or equal to about 75% is released in about 24 hours.

36. Use according to claim 35, wherein less than or equal to about 25% of said N-2,3,3-tetramethylbicyclo[2.1.1]heptan-2-amine is released in about 2 hours, about 20% to about 50% is released in about 4 hours, about 40% to about 70% is released in about 8 hours, greater than or equal to about 55% is released in about 12 hours, and greater than or equal to about 80% is released in about 24 hours.

37. Use according to claim 36, wherein the transdermal formulation further comprises at least one other pharmaceutical active compound.

38. Use according to claim 37, wherein said at least one other pharmaceutically active compound is chosen from ganglionic blockers, nicotinic-receptor antagonists, gastrointestinal motility altering agents, antispasmodics, antimuscarinic agents, opiates, 5-HT receptor agonists, 5-HT receptor antagonists, calcium channel blockers, beta adrenergic receptor blockers, agents that alter fluid transport across the gut, agents that alter fluid transport into or out of gastrointestinal cells, diuretics, anti-diarrheals, H₂-antihistamines, proton pump inhibitors, antacids, anti-inflammatory agents, steroids, mineralocorticoids, corticosteroids, anti-infective agents, immunomodulators, and fish oil.

39. Use according to claim 38, wherein said at least one other pharmaceutically active compound is chosen from hexamethonium, trimethaphan, chloroisondamine, erysodine, β-dihydroerythrodine, amantidine, perpidine, succinylcholine, decamethonium, tubocurarine, atracurium, doxacurium, mivicurium, pancuronium, rocuronium, vencuronium, glycopyrrolate, atropine, hyscomine, scopolamine, loperamide, difenoxine, codeine, morphine, oxymorphone, oxycontin, dihydrocodeine, fentanyl, alosetron hydrochloride, verapamil, amiloride, furosemide, bismuth, sandostatin, sulfasalazine, estrogens, prednisone, prednisolone, cortisol, cortisone, fluticasone, dexamethasone, betamethasone, 5-aminosalicylic acid, metronidazole, ciprofloxacin, azathioprine, 6-mercaptopurine, cyclosporine, methotrexate, fish oil, remicade, heparin, and nicotine.

40. Use according to claim 33, wherein the transdermal formulation comprises racemic N-2,3,3-tetramethylbicyclo-[2.1.1]heptan-2-amine, enriched (R)-N-2,3,3-tetramethylbicyclo-[2.1.1]heptan-2-amine, enriched (S)-N-2,3,3-tetramethylbicyclo-[2.1.1]heptan-2-amine, substantially pure (R)-N-2,3,3-tetramethylbicyclo-[2.1.1]heptan-2-amine, or substantially pure (S)-N-2,3,3-tetramethylbicyclo-[2.1.1]heptan-2-amine, or pharmaceutically acceptable salts thereof.

41. Use according to claim 40, wherein the transdermal formulation comprises racemic N-2,3,3-tetramethylbicyclo-[2.1.1]heptan-2-amine, or pharmaceutically acceptable salts thereof.

42. Use according to claim 40, wherein the transdermal formulation comprises enriched (R)-N-2,3,3-tetramethylbicyclo-[2.1.1]heptan-2-amine, or a pharmaceutically acceptable salt thereof.

43. Use according to claim 40, wherein the transdermal formulation comprises enriched (S)-N-2,3,3-tetramethylbicyclo-[2.1.1]heptan-2-amine, or a pharmaceutically acceptable salt thereof.

## Patentansprüche

1. Verwendung von N-2,3,3-Tetramethylbicyclo-[2.1.1]heptan-2-amin oder eines seiner pharmazeutisch annehmbaren Salze zur Herstellung eines Medikaments zur Reduzierung gastrointestinaler Motilität bei einer Person, die unter einer anormalen Erhöhung der gastrointestinalen Motiliät leidet, wobei das N-2,3,3-Tetramethylbicyclo-[2.1.1]heptan-2-amin, racemisches N-2,3,3-Tetramethylbicyclo-[2.1.1]heptan-2-amin, angereichertes (R)-N-2,3,3-Tetramethylbicyclo-[2.1.1]heptan-2-amin oder angereichertes (S)- N-2,3,3-Tetramethylbicyclo-[2.1.1]heptan-2-amin umfasst, und wobei die anormale Erhöhung der gastrointestinalen Motilität durch wenigstens ein Leiden der folgenden Gruppe verursacht ist: entzündliche Darmerkrankung, ulcerative Colitis, granulomatöse Enteritis, infektiöse Erkrankungen von Dünndarm oder Dickdarm, Spasmen des Pylorus, abdominale Krämpfe, Funktionsstörung des Darmes, milde Dysenterien, Divertikulitis, akute Enterokolitis, neurogene Darmstörungen, Milzflexusyndrom, neurogener Kolon, spastische Kolitis; oder ein Symptom der vorgenannten Leiden.

2. Verwendung nach Anspruch 1, wobei das Leiden eine funktionelle Darmstörung oder reizbares Darmsyndrom ist.

3. Verwendung nach Anspruch 1, wobei bei der Verwendung die anormale Erhöhung der gastrointestinalen Motilität verringert wird, während wenigstens ein mit der Gabe einer üblichen Formulierung von N-2,3,3-Tetramethylbicyclo-[2.1.1]heptan-2-amin oder einem seiner pharmazeutisch annehmbaren Salze verbundener Nebeneffekt minimiert wird.

4. Verwendung nach Anspruch 3, wobei der wenigstens eine Effekt aus den Effekten auf die Pulszahl, Blutdruck, Sehvermögen und Blasenfunktion ausgewählt ist.

5. Verwendung nach Anspruch 1, wobei im Gebrauch das N-2,3,3-Tetramethylbicyclo-[2.1.1]heptan-2-amin oder eines seiner pharmazeutischen annehmbaren Salze in Form einer pharmazeutisch annehmbaren Formulierung verabreicht wird.

6. Verwendung nach Anspruch 5, wobei die pharmazeutisch annehmbare Formulierung eine Formulierung mit modifizierter Freisetzung ist.

7. Verwendung nach Anspruch 5, wobei die pharmazeutisch annehmbare Formulierung eine Formulierung mit modifizierter Freisetzung in Kombination mit einer Formulierung mit sofortiger Freisetzung umfasst.

8. Verwendung nach einem der Ansprüche 5 bis 7, wobei die pharmazeutisch annehmbare Formulierung für orale, intranasale oder transdermale Verabreichung geeignet ist.

9. Verwendung nach Anspruch 8, wobei die pharmazeutisch annehmbare Formulierung für bukkale oder sublinguale Verabreichung geeignet ist.

10. Verwendung nach Anspruch 1, wobei das N-2,3,3-Tetramethylbicyclo-[2.1.1]heptan-2-amin oder eines seiner pharmazeutisch annehmbaren Salze in einer Menge von etwa 0,2 mg bis etwa 40 mg, vorzugsweise in einer Menge von etwa 0,5 mg bis etwa 20 mg, noch weiter bevorzugt in einer Menge von etwa 1 mg bis etwa 15 mg, insbesondere in einer Menge von etwa 2 mg bis etwa 12 mg vorliegt.

11. Verwendung nach Anspruch 1, wobei im Gebrauch die Gabe von N-2,3,3-Tetramethylbicyclo-[2.1.1]heptan-2-amin oder eines seiner pharmazeutisch annehmbaren Salze eine maximale Plasmakonzentration von N-2,3,3-Tetramethylbicyclo-[2.1.1]heptan-2-amin etwa 3,5 Stunden nach einer ersten Gabe oder später, vorzugsweise etwa 6 Stunden nach einer ersten Gabe oder später ergibt.

12. Verwendung nach Anspruch 1, wobei im Gebrauch das N-2,3,3-Tetramethylbicyclo-[2.1.1]heptan-2-amin oder eines seiner pharmazeutisch annehmbaren Salze einmal täglich verabreicht wird.

13. Verwendung nach Anspruch 1, wobei das N-2,3,3-Tetramethylbicyclo-[2.1.1]heptan-2-amin racemisches N-2,3,3-Tetramethylbicyclo-[2.1.1]heptan-2-amin oder eines seiner pharmazeutisch annehmbaren Salze enthält.

14. Verwendung nach Anspruch 1, wobei das N-2,3,3-Tetramethylbicyclo-[2.1.1]heptan-2-amin angereichertes N-2,3,3-Tetramethylbicyclo-[2.1.1]heptan-2-amin oder eines seiner pharmazeutisch annehmbaren Salze enthält.

15. Verwendung nach Anspruch 1, wobei bei der Verwendung das N-2,3,3-Tetramethylbicyclo-[2.1.1]heptan-2-amin oder eines seiner pharmazeutisch annehmbaren Salze in Kombination mit wenigstens einer anderen pharmazeutisch aktiven Verbindung verabreicht wird.

16. Verwendung nach Anspruch 15, wobei die wenigstens eine andere pharmazeutisch aktive Verbindung ausgewählt wird aus: Ganglienblocker, Nikotinrezeptor-Antagonisten, die gastrointestinale Motilität ändernen Mitteln, krampflösenden Mitteln, Mittel gegen Pilzvergiftung, Opiate, 5-HT-Rezeptoragonisten, 5-HT-Rezeptorantagonisten, Kalziumkanalblocker, betaadrenergische Rezeptorblocker, Mittel, die den Flüssigkeitstransport durch den Darm ändern, Mittel, die den Flüssigkeitstransport in die oder aus den gastrointestinalen Zellen ändern, Diuretika, Mittel gegen Diarrhöe, H₂-Antihistamine, Protonenpumpeninhibitoren, Antacide, entzündungshemmende Mittel, Steroide, Mineralocorticoide, Corticosteroide, anti-infektiöse Mittel, Immunmodulatoren und Fischöl.

17. Verwendung nach Anspruch 16, wobei die wenigstens eine andere pharmazeutisch aktive Verbindung ausgewählt ist aus Hexamethonium, Trimetaphan, Chloroisondamin, Erysodin, β-Dihydroerythrodin, Amantidin, Perpidin, Succinylcholin, Decamethonium, Tubocurarin, Atracurium, Doxacurium, Mivicurium, Pancuronium, Rocuronium, Vencuronium, Glycopyrrolat, Atropin, Hyscomin, Scopolamin, Loperamid, Difenoxin, Codein, Morphin, Oxymorphon, Oxycontin, Dihydrocodein, Fentanyl, Alosetronhydrochlorid, Verapamil, Amilorid, Furosemid, Wismuth, Sandostatin, Sulfasalazin, Estrogene, Prednison, Prednisolon, Cortisol, Cortison, Fluticason, Dexamethason, Betamethason, 5-Aminosalicylsäure, Metronidazol, Ciprofloxacin, Azathioprin, 6-Mercaptopurin, Cyclosporin, Methotrexat, Fischöl, Remicade, Heparin und Nikotin.

18. Verwendung nach Anspruch 8, wobei die Formulierung Komponenten mit verlängerter Freisetzung oder Komponenten mit verzögerter Freisetzung oder sowohl Komponenten mit verlängerter Freisetzung als auch mit verzögerter Freisetzung umfasst.

19. Verwendung nach Anspruch 8, wobei bei der Verwendung die Formulierung ein Peak-Tiefpunkt-Plasmaniveau-Verhältnis von N-2,3,3-Tetramethylbicyclo-[2.1.1]heptan-2-amin von weniger als etwa 4:1, vorzugsweise weniger als etwa 3:1, insbesondere weniger als etwa 2:1 erzeugt.

20. Verwendung nach Anspruch 8, wobei die Verabreichung der Formulierung eine Plasmakonzentration von N-2,3,3-Tetramethylbicyclo-[2.1.1]heptan-2-amin wenigstens etwa 24 Stunden nach der ersten Gabe erzeugt, die etwa 25 % von der Peak-Plasmakonzentration größer oder gleich ist, die nach der Gabe erreicht wird, vorzugsweise, wobei die Plasmakonzentration von N-2,3,3-Tetramethylbicyclo-[2.1.1]heptan-2-amin wenigstens etwa 24 Stunden nach einer ersten Gabe größer als oder gleich wie etwa 50 % der Peak-Plasmakonzentration ist, die nach der Gabe erreicht wird.

21. Verwendung nach Anspruch 8, wobei die Verabreichung der Formulierung eine Plasmakonzentration von N-2,3,3-Tetramethylbicyclo-[2,1.1]heptan-2-amin, die größer als oder gleich wie etwa 50 % der Peak-Plasmakonzentration ist für mehr als oder gleich etwa 14 Stunden nach der ersten Verabreichung, vorzugsweise größer als oder gleich wie etwa 50 % der Peak-Plasmakonzentration ist für mehr als oder gleich etwa 16 Stunden nach einer ersten Verabreichung.

22. Verwendung nach Anspruch 21, wobei die Plasmakonzentration von N-2,3,3-Tetramethylbicyclo-[2.1.1]heptan-2-amin größer als oder gleich wie etwa 50 % der Peak-Plasmakonzentration für mehr als oder gleich etwa 18 Stunden nach einer ersten Verabreichung, vorzugsweise größer als oder gleich wie etwa 50 % der Peak-Plasmakonzentration für mehr als oder gleich etwa 24 Stunden nach einer ersten Verabreichung ist.

23. Verwendung nach Anspruch 8, wobei die Formulierung bei einem Test in einem U.S. Pharmacopeia (USP) Typ 2-Apparat bei 37°C, bei 50 Upm gerührt und in einem Phosphatpuffer vom pH 6,8 weniger als etwa 50 % des N-2,3,3-Tetramethylbicyclo-[2.1.1]heptan-2-amin in weniger als 2 Stunden, mehr als oder gleich wie etwa 40 % in etwa 12 oder mehr Stunden und etwa 70 % oder mehr in etwa 24 oder mehr Stunden freisetzt.

24. Verwendung nach Anspruch 23, wobei weniger als oder gleich wie etwa 50 % des N-2,3,3-Tetramethylbicyclo-[2.1.1]heptan-2-aminin in etwa 2 Stunden, weniger als oder gleich etwa 70 % in etwa 4 Stunden, mehr als oder gleich etwa 50 % in etwa 8 Stunden, mehr als oder gleich wie etwa 65 % in etwa 12 Stunden und mehr als oder gleich etwa 80 % in etwa 24 Stunden freigesetzt werden.

25. Verwendung nach Anspruch 24, wobei weniger als oder gleich wie etwa 40 % des N-2,3,3-Tetramethylbicyclo-[2.1.1]heptan-2-amin in etwa 2 Stunden, weniger als oder gleich wie etwa 65 % in etwa 4 Stunden, mehr als oder gleich wie etwa 60 % in etwa 8 Stunden, mehr als oder gleich wie etwa 70 % in etwa 12 Stunden und mehr als oder gleich wie etwa 80 % in etwa 24 Stunden freigesetzt werden.

26. Verwendung nach Anspruch 25, wobei weniger als oder gleich wie etwa 30 % des N-2,3,3-Tetramethylbicyclo-[2.1.1]heptan-2-amin in etwa 2 Stunden, etwa 20 bis etwa 60 % in etwa 4 Stunden, mehr als oder gleich wie etwa 70 % in etwa 8 Stunden, mehr als oder gleich wie etwa 75 % in etwa 12 Stunden und mehr als oder gleich wie etwa 80 % in etwa 24 Stunden freigesetzt werden.

27. Verwendung nach Anspruch 8, wobei die Formulierung racemisches N-2,3,3-Tetramethylbicyclo-[2.1.1]heptan-2-amin oder eines seiner pharmazeutisch annehmbaren Salze enthält.

28. Verwendung nach Anspruch 8, wobei die Formulierung angereichtertes (R)-N-2,3,3-Tetramethylbicyclo-[2.1.1]heptan-2-amin oder eines seiner pharmazeutisch annehmbaren Salze enthält.

29. Verwendung nach Anspruch 8, wobei die Formulierung angereichertes (S)-N-2,3,3-Tetramethylbicyclo-[2.1.1]heptan-2-amin oder eines seiner pharmazeutisch annehmbaren Salze enthält.

30. Verwendung nach Anspruch 8, wobei die Formulierung wenigstens eine andere pharmazeutisch aktive Verbindung enthält.

31. Verwendung nach Anspruch 30, wobei die wenigstens eine andere pharmazeutische aktive Verbindung ausgewählt ist aus: Ganglienblocker, Nikotinrezeptor-Antagonisten, die gastrointestinale Motilität ändernen Mitteln, krampflösenden Mitteln, Mittel gegen Pilzvergiftung, Opiate, 5-HT-Rezeptoragonisten, 5-HT-Rezeptorantagonisten, Kalziumkanalblocker, betaadrenergische Rezeptorblocker, Mittel, die den Flüssigkeitstransport durch den Darm ändern, Mittel, die den Flüssigkeitstransport in die oder aus den gastrointestinalen Zellen ändern, Diuretika, Mittel gegen Diarrhöe, H₂-Antihistamine, Protonenpumpinhibitoren, Antacide, entzündungshemmende Mittel, Steroide, Mineralocorticoide, Corticosteroide, anti-infektiöse Mittel, Immunmodulatoren und Fischöl.

32. Verwendung nach Anspruch 31, wobei die wenigstens eine andere pharmazeutisch aktive Verbindung ausgewählt ist aus Hexamethonium, Trimetaphan, Chloroisondamin, Erysodin, β-Dihydroerythrodin, Amantidin, Perpidin, Succinylcholin, Decamethonium, Tubocurarin, Atracurium, Doxacurium, Mivicurium, Pancuronium, Rocuronium, Vencuronium, Glycopyrrolat, Atropin, Hyscomin, Scopolamin, Loperamid, Difenoxin, Codein, Morphin, Oxymorphon, Oxycontin, Dihydrocodein, Fentanyl, Alosetronhydrochlorid, Verapamil, Amilorid, Furosemid, Wismuth, Sandostatin, Sulfasalazin, Estrogene, Prednison, Prednisolon, Cortisol, Cortison, Fluticason, Dexamethason, Betamethason, 5-Aminosalicylsäure, Metronidazol, Ciprofloxacin, Azathioprin, 6-Mercaptopurin, Cyclosporin, Methotrexat, Fischöl, Remicade, Heparin und Nikotin.

33. Verwendung nach Anspruch 5, wobei die Formulierung eine transdermale Formulierung ist, die beim Test unter Verwendung von modifizierten Franz-Diffusionszellen von menschlicher Epidermis in Phosphatpuffer bei einem pH von etwa 4 bis etwa 7 bei etwa 32°C eine Permeationsrate aufweist, bei der weniger als etwa 50% des N-2,3,3-Tetramethylbicyclo-[2.1.1]heptan-2-amin in weniger als.etwa 2 Stunden, mehr als oder gleich etwa 40% in etwa 12 Stunden oder mehr und etwa 70 % oder mehr in etwa 24 Stunden oder mehr freigesetzt werden.

34. Verwendung nach Anspruch 33, wobei die mehr als oder gleich etwa 40 % des N-2,3,3-Tetramethylbicyclo-[2.1.1]heptan-2-amin in etwa 2 Stunden, etwa 10 % bis etwa 70 % in etwa 4 Stunden, etwa 20 % bis etwa 80 % in etwa 8 Stunden, mehr als oder gleich wie etwa 40 % in etwa 12 Stunden und mehr als oder gleich wie etwa 70 % in etwa 24 Stunden freigesetzt werden.

35. Verwendung nach Anspruch 34, wobei weniger als oder gleich wie etwa 30 % des N-2,3,3-Tetramethylbicyclo-[2.1.1]heptan-2-amin in etwa 2 Stunden, etwa 15 bis etwa 60 % in etwa 4 Stunden, etwa 30 bis etwa 70 % in etwa 8 Stunden, mehr als oder gleich wie etwa 50 % in etwa 12 Stunden und mehr als oder gleich wie etwa 75 % in etwa 24 Stunden freigesetzt werden.

36. Verwendung nach Anspruch 35, wobei weniger als oder gleich wie etwa 25 % des N-2,3,3-Tetramethylbicyclo-[2.1.1]heptan-2-amin in etwa 2 Stunden, etwa 20 bis etwa 50 % in etwa 4 Stunden, etwa 40 bis etwa 70 % in etwa 8 Stunden, mehr als oder gleich wie etwa 55 % in etwa 12 Stunden und mehr als oder gleich wie etwa 80 % in etwa 24 Stunden freigesetzt werden.

37. Verwendung nach Anspruch 36, wobei die transdermale Formulierung weiterhin wenigstens eine andere pharmazeutische aktive Verbindung enthält.

38. Verwendung nach Anspruch 37, wobei die wenigstens eine andere pharmazeutisch aktive Verbindung ausgewählt ist aus: Ganglienblocker, Nikotinrezeptor-Antagonisten, die gastrointestinale Motilität ändernen Mitteln, krampflösenden Mitteln, Mittel gegen Pilzvergiftung, Opiate, 5-HT-Rezeptoragonisten, 5-HT-Rezeptorantagonisten, Kalziumkanalblocker, betaadrenergische Rezeptorblocker, Mittel, die den Flüssigkeitstransport durch den Darm ändern, Mittel, die den Flüssigkeitstransport in die oder aus den gastrointestinalen Zellen ändern, Diuretika, Mittel gegen Diarrhöe, H₂-Antihistamine, Protonenpumpeninhibitoren, Antacide, entzündungshemmende Mittel, Steroide, Mineralocorticoide, Corticosteroide, anti-infektiöse Mittel, Immunmodulatoren und Fischöl.

39. Verwendung nach Anspruch 38, wobei die wenigstens eine andere pharmazeutisch aktive Verbindung ausgewählt ist aus Hexamethonium, Trimetaphan, Chloroisondamin, Erysodin, β-Dihydroerythrodin, Amantidin, Perpidin, Succinylcholin, Decamethonium, Tubocurarin, Atracurium, Doxacurium, Mivicurium, Pancuronium, Rocuronium, Vencuronium, Glycopyrrolat, Atropin, Hyscomin, Scopolamin, Loperamid, Difenoxin, Codein, Morphin, Oxymorphon, Oxycontin, Dihydrocodein, Fentanyl, Alosetronhydrochlorid, Verapamil, Amilorid, Furosemid, Wismuth, Sandostatin, Sulfasalazin, Estrogene, Prednison, Prednisolon, Cortisol, Cortison, Fluticason, Dexamethason, Betamethason, 5-Aminosalicylsäure, Metronidazol, Ciprofloxacin, Azathioprin, 6-Mercaptopurin, Cyclosporin, Methotrexat, Fischöl, Remicade, Heparin und Nikotin.

40. Verwendung nach Anspruch 33, wobei die transdermale Formulierung racemisches N-2,3,3-Tetramethylbicyclo-[2.1.1]heptan-2-amin, angereichertes (R)-N-2,3,3-Tetramethylbicyclo-[2.1.1]heptan-2-amin, angereichertes (S)-N-2,3,3-Tetramethylbicyclo-[2.1.1]heptan-2-amin, im Wesentlichen reines (R)-N-2,3,3-Tetramethylbicyclo-[2.1.1]heptan-2-amin oder im Wesentlichen reines (S)-N-2,3,3-Tetramethylbicyclo-[2.1.1]heptan-2-amin oder eines seiner pharmazeutisch annehmbaren Salze enthält.

41. Verwendung nach Anspruch 40, wobei die transdermale Formulierung racemisches N-2,3,3-Tetramethylbicyclo-[2.1.1]heptan-2-amin oder eines seiner pharmazeutisch annehmbaren Salze enthält.

42. Verwendung nach Anspruch 40, wobei die transdermale Formulierung angereichertes (R)-N-2,3,3-Tetramethylbicyclo-[2.1.1]heptan-2-amin oder eines seiner pharmazeutisch annehmbaren Salze enthält.

43. Verwendung nach Anspruch 40, wobei die transdermale Formulierung angereichertes (S)-N-2,3,3-Tetramethylbicyclo-[2.1.1]heptan-2-amin oder eines seiner pharmazeutisch annehmbaren Salze enthält.

## Revendications

1. Utilisation de la N-2,3,3-tétraméthylbicyclo-[2.1.1]heptan-2-amine ou de l'un de ses sels pharmaceutiquement acceptables pour la fabrication d'un médicament destiné à réduire la motilité gastro-intestinale chez un sujet qui présente une augmentation anormale de la motilité gastro-intestinale, ladite N-2,3,3-tétraméthylbicyclo-[2.1.1]heptan-2-amine comprenant une N-2,3,3-tétraméthylbicyclo-[2.1.1]heptan-2-amine racémique, une (R)-N-2,3,3-tétraméthylbicyclo-[2.1.1]heptan-2-amine enrichie, ou une (S)-N-2,3,3-tétraméthylbicyclo-[2.1.1]heptan-2-amine enrichie, et ladite augmentation anormale de la motilité gastro-intestinale étant causée par au moins un état choisi parmi la maladie inflammatoire intestinale, la rectocolite hémorragique, l'entérite régionale, les maladies infectieuses du gros intestin ou de l'intestin grêle, le spasme du pylore, les crampes abdominales, un trouble intestinal fonctionnel, les dysenteries bénignes, la diverticulite, l'entérocolite aiguë, les troubles intestinaux d'origine nerveuse, le syndrome de l'angle splénique, le côlon neurogène, la colite spasmodique ou étant un symptôme de l'un quelconque des états précédents.

2. Utilisation selon la revendication 1, dans laquelle l'état est un trouble intestinal fonctionnel ou un syndrome de côlon irritable.

3. Utilisation selon la revendication 1, dans laquelle lors de l'utilisation, ladite augmentation anormale de la motilité gastro-intestinale est réduite tout en minimisant au moins un effet secondaire associé à l'administration d'une formulation conventionnelle de N-2,3,3-tétraméthylbicyclo-[2.1.1]heptan-2-amine ou de l'un de ses sels pharmaceutiquement acceptables.

4. Utilisation selon la revendication 3, dans laquelle ledit ou lesdits effets sont choisis parmi des effets sur la fréquence cardiaque, la pression sanguine, la vision et la fonction de la vessie dudit sujet.

5. Utilisation selon la revendication 1, dans laquelle lors de l'utilisation, ladite N-2,3,3-tétraméthylbicyclo[2.1.1]heptan-2-amine ou un de ses sels pharmaceutiquement acceptables est administré sous la forme d'une formulation pharmaceutiquement acceptable.

6. Utilisation selon la revendication 5, dans laquelle ladite formulation pharmaceutiquement acceptable est une formulation à libération modifiée.

7. Utilisation selon la revendication 5, dans laquelle ladite formulation pharmaceutiquement acceptable comprend une formulation à libération modifiée en combinaison avec une formulation à libération immédiate.

8. Utilisation selon l'une quelconque des revendications 5 à 7, dans laquelle ladite formulation pharmaceutiquement acceptable convient pour une administration orale, intra-nasale ou transdermique.

9. Utilisation selon la revendication 8, dans laquelle ladite formulation pharmaceutiquement acceptable convient pour une administration buccale ou sublinguale.

10. Utilisation selon la revendication 1, dans laquelle ladite N-2,3,3-tétraméthylbicylo-[2.1.1]heptan-2-amine ou un de ses sels pharmaceutiquement acceptables est présent en une quantité d'environ 0,2 mg à environ 40 mg, de préférence en une quantité d'environ 0,5 mg à environ 20 mg, de manière encore préférable en une quantité d'environ 1 mg à environ 15 mg, de manière particulièrement préférable en une quantité d'environ 2 mg à environ 12 mg.

11. Utilisation selon la revendication 1, dans laquelle lors de l'utilisation, l'administration de la N-2,3,3-tétraméthylbicyclo-[2.1.1]heptan-2-amine ou de l'un de ses sels pharmaceutiquement acceptables fournit une concentration plasmatique maximale de N-2,3,3-tétraméthylbicyclo[2.1.1]heptan-2-amine à environ 3,5 heures ou plus tard à la suite d'une première administration, de préférence à environ 6 heures ou plus tard à la suite d'une première administration.

12. Utilisation selon la revendication 1, dans laquelle lors de l'utilisation, ladite N-2,3,3-tétraméthylbicyclo[2.1.1]heptan-2-amine ou un de ses sels pharmaceutiquement acceptables est administré une fois par jour.

13. Utilisation selon la revendication 1, dans laquelle ladite N-2,3,3-tétraméthylbicyclo-[2.1.1]heptan-2-amine comprend une N-2,3,3-tétraméthylbicyclo-[2.1.1]heptan-2-amine racémique ou un de ses sels pharmaceutiquement acceptables.

14. Utilisation selon la revendication 1, dans laquelle ladite N-2,3,3-tétraméthylbicyclo-[2.1.1]heptan-2-amine comprend une N-2,3,3-tétraméthylbicyclo-[2.1.1]heptan-2-amine enrichie ou un de ses sels pharmaceutiquement acceptables.

15. Utilisation selon la revendication 1, dans laquelle lors de l'utilisation, ladite N-2,3,3-tétraméthylbicyclo[2.1.1]heptan-2-amine ou un de ses sels pharmaceutiquement acceptables est administré en combinaison avec au moins un autre composé pharmaceutiquement actif.

16. Utilisation selon la revendication 15, dans laquelle ledit ou lesdits autres composés pharmaceutiquement actifs sont choisis parmi des agents bloquants ganglionnaires, des antagonistes des récepteurs nicotiniques, des agents modificateurs de la motilité gastro-intestinale, des antispasmodiques, des agents antimuscariniques, des opiacés, des agonistes des récepteurs de 5-HT, des antagonistes des récepteurs de 5-HT, des agents bloquants des canaux calciques, des agents bloquants des récepteurs bêta-adrénergiques, des agents modificateurs du transport des fluides dans l'intestin, des agents modificateurs du transport des fluides entrant ou sortant des cellules gastro-intestinales, des diurétiques, des antidiarrhéiques, des inhibiteurs des récepteurs H₂ de l'histamine, des inhibiteurs de la pompe à protons, des antiacides, des agents anti-inflammatoires, des stéroïdes, des minéralocorticoïdes, des corticostéroïdes, des agents anti-infectieux, des immunomodulateurs et l'huile de poisson.

17. Utilisation selon la revendication 16, dans laquelle ledit ou lesdits autres composés pharmaceutiquement actifs sont choisis parmi l'hexaméthonium, le trimétaphan, la chlorisondamine, l'érysodine, la β-dihydroérythrodine, l'amantidine, la perpidine, la succinylcholine, le décaméthonium, la tubocurarine, l'atracurium, le doxacurium, le mivicurium, le pancuronium, le rocuronium, le vencuronium, le glycopyrrolate, l'atropine, l'hyscomine, la scopolamine, le lopéramide, la difénoxine, la codéine, la morphine, l'oxymorphone, l'oxycontin, la dihydrocodéine, le fentanyl, le chlorhydrate d'alosétron, le vérapamil, l'amiloride, le furosémide, le bismuth, la sandostatine, la sulfasalazine, les estrogènes, la prednisone, la prednisolone, le cortisol, la cortisone, la fluticasone, la dexaméthasone, la bêtaméthasone, l'acide 5-aminosalicylique, le métronidazole, la ciprofloxacine, l'azathioprine, la 6-mercaptopurine, la cyclosporine, le méthotrexate, l'huile de poisson, le remicade, l'héparine et la nicotine.

18. Utilisation selon la revendication 8, dans laquelle ladite formulation comprend des composants à libération prolongée, des composants à libération retardée ou à la fois des composants à libération prolongée et des composants à libération retardée.

19. Utilisation selon la revendication 8, dans laquelle lors de l'utilisation, ladite formulation produit un rapport des taux plasmatiques maximum/minimum de la N-2,3,3-tétraméthylbicyclo-[2.1.1]heptan-2-amine inférieur à environ 4/1, de préférence inférieur à environ 3/1, de manière encore préférable inférieur à environ 2/1.

20. Utilisation selon la revendication 8, dans laquelle l'administration de ladite formulation fournit une concentration plasmatique de la N-2,3,3-tétraméthylbicyclo[2.1.1]heptan-2-amine, au moins environ 24 heures à la suite d'une première administration, qui est supérieure ou égale à environ 25 % de la concentration plasmatique maximale obtenue à la suite de ladite administration, de préférence dans laquelle ladite concentration plasmatique de la N-2,3,3-tétraméthylbicyclo[2.1.1]heptan-2-amine, au moins environ 24 heures à la suite d'une première administration, est supérieure ou égale à environ 50 % de la concentration plasmatique maximale obtenue après ladite administration.

21. Utilisation selon la revendication 8, dans laquelle l'administration de ladite formulation fournit une concentration plasmatique de la N-2,3,3-tétraméthylbicyclo-[2.1.1]heptan-2-amine qui est supérieure ou égale à environ 50 % de la concentration plasmatique maximale, pendant une durée supérieure ou égale à environ 14 heures, à la suite d'une première administration, de préférence supérieure ou égale à environ 50 % de la concentration plasmatique maximale, pendant une durée supérieure ou égale à environ 16 heures, à la suite d'une première administration.

22. Utilisation selon la revendication 21, dans laquelle ladite concentration plasmatique de la N-2,3,3-tétraméthylbicyclo-[2.1.1]heptan-2-amine est supérieure ou égale à environ 50 % de la concentration plasmatique maximale, pendant une durée supérieure ou égale à environ 18 heures, à la suite d'une première administration, de préférence supérieure ou égale à environ 50 % de la concentration plasmatique maximale, pendant une durée supérieure ou égale à environ 24 heures, à la suite d'une première administration.

23. Utilisation selon la revendication 8, dans laquelle la formulation, lorsqu'elle est testée dans un appareil de type 2 selon la pharmacopée américaine (U.S. Pharmacopeia ; USP) à 37 °C, agitée à 50 t/min et dans un tampon phosphate à pH 6,8, libère moins de 50 % environ de ladite N-2,3,3-tétraméthylbicyclo[2.1.1]heptan-2-amine en moins de 2 heures environ, environ 40 % ou plus, en environ 12 heures ou plus ; et environ 70 % ou plus, en environ 24 heures ou plus.

24. Utilisation selon la revendication 23, dans laquelle environ 50 % ou moins de ladite N-2,3,3-tétraméthylbicyclo[2.1.1]heptan-2-amine sont libérés en environ 2 heures, environ 70 % ou moins sont libérés en environ 4 heures, environ 50 % ou plus sont libérés en environ 8 heures, environ 65 % ou plus sont libérés en environ 12 heures ; et environ 80 % ou plus sont libérés en environ 24 heures.

25. Utilisation selon la revendication 24, dans laquelle environ 40 % ou moins de ladite N-2,3,3-tétraméthylbicyclo[2.1.1]heptan-2-amine sont libérés en environ 2 heures, environ 65 % ou moins sont libérés en environ 4 heures, environ 60 % ou plus sont libérés en environ 8 heures, environ 70 % ou plus sont libérés en environ 12 heures et environ 80 % ou plus sont libérés en environ 24 heures.

26. Utilisation selon la revendication 25, dans laquelle environ 30 % ou moins de ladite N-2,3,3-tétraméthylbicyclo[2.1.1]heptan-2-amine sont libérés en environ 2 heures, d'environ 20 % à environ 60 % sont libérés en environ 4 heures, environ 70 % ou plus sont libérés en environ 8 heures, environ 75 % ou plus sont libérés en environ 12 heures et environ 80 % ou plus sont libérés en environ 24 heures.

27. Utilisation selon la revendication 8, dans laquelle ladite formulation comprend une N-2,3,3-tétraméthylbicyclo-[2.1.1]heptan-2-amine racémique ou un de ses sels pharmaceutiquement acceptables.

28. Utilisation selon la revendication 8, dans laquelle ladite formulation comprend une (R)-N-2,3,3-tétraméthylbicyclo-[2.1.1]heptan-2-amine enrichie ou un de ses sels pharmaceutiquement acceptables.

29. Utilisation selon la revendication 8, dans laquelle ladite formulation comprend une (S)-N-2,3,3-tétraméthylbicyclo-[2.1.1]heptan-2-amine enrichie ou un de ses sels pharmaceutiquement acceptables.

30. Utilisation selon la revendication 8, dans laquelle ladite formulation comprend au moins un autre composé pharmaceutiquement actif.

31. Utilisation selon la revendication 30, dans laquelle ledit ou lesdits autres composés pharmaceutiquement actifs sont choisis parmi des agents bloquants ganglionnaires, des antagonistes des récepteurs nicotiniques, des agents modificateurs de la motilité gastro-intestinale, des antispasmodiques, des agents antimuscariniques, des opiacés, des agonistes des récepteurs de 5-HT, des antagonistes des récepteurs de 5-HT, des agents bloquants des canaux calciques, des agents bloquants des récepteurs bêta-adrénergiques, des agents modificateurs du transport des liquides dans l'intestin, des agents modificateurs du transport des liquides entrant ou sortant des cellules gastro-intestinales, des diurétiques, des antidiarrhéiques, des inhibiteurs des récepteurs H₂ de l'histamine, des inhibiteurs de la pompe à protons, des antiacides, des agents anti-inflammatoires, des stéroïdes, des minéralocorticoïdes, des corticostéroïdes, des agents anti-infectieux, des immunomodulateurs et l'huile de poisson.

32. Utilisation selon la revendication 31, dans laquelle ledit ou lesdits autres composés pharmaceutiquement actifs sont choisis parmi l'hexaméthonium, le trimétaphan, la chlorisondamine, l'érysodine, la p-dihydroérythrodine, l'amantidine, la perpidine, la succinylcholine, le décaméthonium, la tubocurarine, l'atracurium, le doxacurium, le mivicurium, le pancuronium, le rocuronium, le vencuronium, le glycopyrrolate, l'atropine, l'hyscomine, la scopolamine, le lopéramide, la difénoxine, la codéine, la morphine, l'oxymorphone, l'oxycontin, la dihydrocodéine, le fentanyl, le chlorhydrate d'alosétron, le vérapamil, l'amiloride, le furosémide, le bismuth, la sandostatine, la sulfasalazine, les estrogènes, la prednisone, la prednisolone, le cortisol, la cortisone, la fluticasone, la dexaméthasone, la bêtaméthasone, l'acide 5-aminosalicylique, le métronidazole, la ciprofloxacine, l'azathioprine, la 6-mercaptopurine, la cyclosporine, le méthotrexate, l'huile de poisson, le remicade, l'héparine et la nicotine.

33. Utilisation selon la revendication 5, dans laquelle la formulation est une formulation transdermique qui, quand elle est testée à l'aide de cellules de diffusion de Franz modifiées à épiderme humain, dans un tampon phosphate à un pH d'environ 4 à environ 7, à environ 32 °C, présente un taux de perméation dans lequel moins de 50 % environ de la N-2,3,3-tétraméthylbicyclo[2.1.1]heptan-2-amine sont libérés en moins de 2 heures environ, environ 40 % ou plus sont libérés en environ 12 heures ou plus et environ 70 % ou plus sont libérés en environ 24 heures ou plus.

34. Utilisation selon la revendication 33, dans laquelle environ 40 % ou moins de ladite N-2,3,3-tétraméthylbicyclo[2.1.1]heptan-2-amine sont libérés en environ 2 heures, d'environ 10 % à environ 70% sont libérés en environ 4 heures, d'environ 20 % à environ 80 % sont libérés en environ 8 heures, environ 40 % ou plus sont libérés en environ 12 heures et environ 70 % ou plus sont libérés en environ 24 heures.

35. Utilisation selon la revendication 34, dans laquelle environ 30 % ou moins de ladite N-2,3,3-tétraméthylbicyclo[2.1.1]heptan-2-amine sont libérés en environ 2 heures, d'environ 15 % à environ 60 % sont libérés en environ 4 heures, d'environ 30 % à environ 70 % sont libérés en environ 8 heures, environ 50 % ou plus sont libérés en environ 12 heures et environ 75 % ou plus sont libérés en environ 24 heures.

36. Utilisation selon la revendication 35, dans laquelle environ 25 % ou moins de ladite N-2,3,3-tétraméthylbicyclo[2.1.1]heptan-2-amine sont libérés en environ 2 heures, d'environ 20 % à environ 50 % sont libérés en environ 4 heures, d'environ 40 % à environ 70 % sont libérés en environ 8 heures, environ 55 % ou plus sont libérés en environ 12 heures et environ 80 % ou plus sont libérés en environ 24 heures.

37. Utilisation selon la revendication 36, dans laquelle la formulation transdermique comprend en outre au moins un autre composé pharmaceutiquement actif.

38. Utilisation selon la revendication 37, dans laquelle ledit ou lesdits autres composés pharmaceutiquement actifs sont choisis parmi des agents bloquants ganglionnaires, des antagonistes des récepteurs nicotiniques, des agents modificateurs de la motilité gastro-intestinale, des antispasmodiques, des agents antimuscariniques, des opiacés, des agonistes des récepteurs de 5-HT, des antagonistes des récepteurs de 5-HT, des agents bloquants des canaux calciques, des agents bloquants des récepteurs bêta-adrénergiques, des agents modificateurs du transport des liquides dans l'intestin, des agents modificateurs du transport des liquides entrant ou sortant des cellules gastro-intestinales, des diurétiques, des antidiarrhéiques, des inhibiteurs des récepteurs H₂ de l'histamine, des inhibiteurs de la pompe à protons, des antiacides, des agents anti-inflammatoires, des stéroïdes, des minéralocorticoïdes, des corticostéroïdes, des agents anti-infectieux, des immunomodulateurs et l'huile de poisson.

39. Utilisation selon la revendication 38, dans laquelle ledit ou lesdits autres composés pharmaceutiquement actifs sont choisis parmi l'hexaméthonium, le trimétaphan, la chlorisondamine, l'érysodine, p-dihydroérythrodine, l'amantidine, la perpidine, la succinylcholine, le décaméthonium, la tubocurarine, l'atracurium, le doxacurium, le mivicurium, le pancuronium, le rocuronium, le vencuronium, le glycopyrrolate, l'atropine, l'hyscomine, la scopolamine, le lopéramide, la difénoxine, la codéine, la morphine, l'oxymorphone, l'oxycontin, la dihydrocodéine, le fentanyl, le chlorhydrate d'alosétron, le vérapamil, l'amiloride, le furosémide, le bismuth, la sandostatine, la sulfasalazine, les estrogènes, la prednisone, la prednisolone, le cortisol, la cortisone, la fluticasone, la dexaméthasone, la bêtaméthasone, l'acide 5-aminosalicylique, le métronidazole, la ciprofloxacine, l'azathioprine, la 6-mercaptopurine, la cyclosporine, le méthotrexate, l'huile de poisson, le remicade, l'héparine et la nicotine.

40. Utilisation selon la revendication 33, dans laquelle la formulation transdermique comprend la N-2,3,3-tétraméthylbicyclo-[2.1.1]heptan-2-amine racémique, la (R)-N-2,3,3-tétraméthylbicyclo-[2.1.1]heptan-2-amine enrichie, la (S)-N-2,3,3-tétraméthylbicyclo-[2.1.1]heptan-2-amine enrichie, la (R)-N-2,3,3-tétraméthylbicyclo-[2.1.1]heptan-2-amine pratiquement pure, la (S)-N-2,3,3-tétraméthylbicyclo-[2.1.1]heptan-2-amine pratiquement pure ou leurs sels pharmaceutiquement acceptables.

41. Utilisation selon la revendication 40, dans laquelle la formulation transdermique comprend la N-2,3,3-tétraméthylbicyclo-[2.1.1]heptan-2-amine racémique ou ses sels pharmaceutiquement acceptables.

42. Utilisation selon la revendication 40, dans laquelle la formulation transdermique comprend la (R)-N-2,3,3-tétraméthylbicyclo-[2.1.1]heptan-2-amine enrichie ou un de ses sels pharmaceutiquement acceptables.

43. Utilisation selon la revendication 40, dans laquelle la formulation transdermique comprend la (S)-N-2,3,3-tétraméthylbicyclo-[2.1.1]heptan-2-amine enrichie ou un de ses sels pharmaceutiquement acceptables.
